(19) Europäisches Patentamt
European Patent Office
Office européen des brevets

(11) Veröffentlichungsnummer : **0 194 416 B1**

(12) **EUROPÄISCHE PATENTSCHRIFT**

(45) Veröffentlichungstag der Patentschrift : **26.04.89**

(21) Anmeldenummer : **86100656.7**

(22) Anmeldetag : **20.01.86**

(51) Int. Cl.[4] : **C 07 D495/14**, A 61 K 31/55// C07D333/36, C07D495/04 ,(C07D495/14, 333:00, 249:00, 243:00)

(54) **Thieno-triazolo-1,4-diazepino-2-carbonsäureamide, Verfahren zur ihrer Herstellung und pharmazeutische Zusammensetzung.**

(30) Priorität : **25.01.85 DE 3502392**

(43) Veröffentlichungstag der Anmeldung : **17.09.86 Patentblatt 86/38**

(45) Bekanntmachung des Hinweises auf die Patenterteilung : **26.04.89 Patentblatt 89/17**

(84) Benannte Vertragsstaaten : **AT BE CH DE FR GB IT LI LU NL SE**

(56) Entgegenhaltungen : **FR-A- 2 217 007**

(73) Patentinhaber : **BOEHRINGER INGELHEIM KG**
**Postfach 200**
**D-6507 Ingelheim am Rhein (DE)**
**BE CH DE FR IT LI LU NL SE AT**
**BOEHRINGER INGELHEIM INTERNATIONAL G.M.B.H.**
**Postfach 20**
**D-6507 Ingelheim am Rhein (DE)**
**GB**

(72) Erfinder : **Weber, Karl-Heinz, Dr.**
**Kaiser-Karl-Strasse 11**
**D-6535 Gau-Algesheim (DE)**
Erfinder : **Walther, Gerhard, Dr.**
**Pfarrer-Heberer-Strasse 37**
**D-6530 Bingen/Rh. (DE)**
Erfinder : **Harreus, Albrecht, Dr.**
**Sandstrasse 1**
**D-6507 Ingelheim/Rh. (DE)**
Erfinder : **Casals-Stenzel, Jorge, Dr.**
**Sertoriusring 295**
**D-6500 Mainz (DE)**
Erfinder : **Muacevic, Gojki, Dr.**
**In der Dörrwiese 13**
**D-6507 Ingelheim/Rh. (DE)**
Erfinder : **Tröger, Wolfgang, Dr.**
**St. Jakobus-Strasse 25**
**D-6534 Stromberg (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 194 416 B1

Jouve, 18, rue St-Denis, 75001 Paris, France

**Beschreibung**

Die Erfindung betrifft neue Thieno-triazolo-1,4-diazepino-2-carbonsäureamide der allgemeinen Formel I

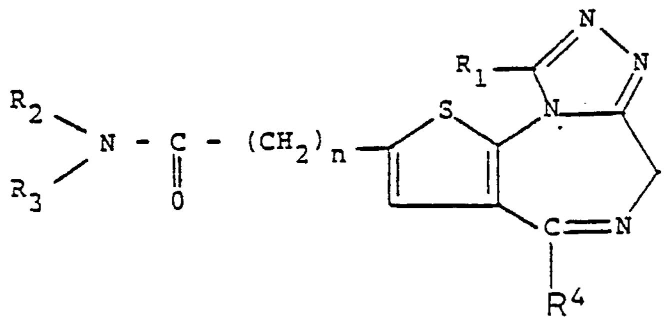

(I)

In dieser Formel bedeuten :

$R_1$ Wasserstoff, eine geradkettige oder verzweigte Alkylgruppe mit 1-4 Kohlenstoffatomen, gegebenenfalls durch Halogen, bevorzugt Cl oder Br. oder Hydroxy substituiert, Cyclopropyl, Alkoxy mit 1-3 Kohlenstoffatomen, bevorzugt Methoxy, Halogen, bevorzugt Chlor oder Brom ;

$R_2$ und $R_3$, die gleich oder verschieden sein können, Wasserstoff, einen geradkettigen oder verzweigten Alkyl- oder Hydroxyalkylrest mit 1-4 Kohlenstoffatomen oder beide Reste $R_2$ und $R_3$ zusammen mit dem Stickstoffatom einen 5-, 6- oder 7-Ring, der gegebenenfalls als weiteres Heteroatom ein Stickstoff-, Sauerstoff- oder Schwefelatom enthalten und wobei das zweite Stickstoffatom gegebenenfalls durch eine Alkylgruppe mit 1-4 Kohlenstoffatomen, bevorzugt die Methylgruppe, substituiert sein kann ;

$R_4$ Phenyl, wobei der Phenylring, bevorzugt in 2-Stellung, durch Methyl, Halogen, besonders bevorzugt Chlor oder Brom, Nitro oder Trifluormethyl substituiert sein kann oder α-Pyridyl ; und

n eine der Zahlen 0, 1, 2, 3, 4, 5, 6, 7 oder 8.

Soweit nichts anderes angegeben, bedeutet Halogen eines der Halogenatome Fluor, Chlor, Brom oder Jod.

Bevorzugt sind Verbindungen der allgemeinen Formel I, in denen n eine der Zahlen 0, 1, 2 bedeuten, besonders bevorzugt solche, in denen n = 2 ist.

Bevorzugte Alkylgruppen sind Methyl, Ethyl, Propyl, iso-Propyl, Butyl und tert.-Butyl.

Die Erfindung umfaßt ferner Verfahren zur Herstellung der neuen Verbindungen und pharmazeutische Zusammensetzungen, die diese Verbindungen als Wirkstoffe enthalten.

Die neuen Verbindungen können in üblicher Weise aus den entsprechenden 2-Carbonsäuren der allgemeinen Formel II

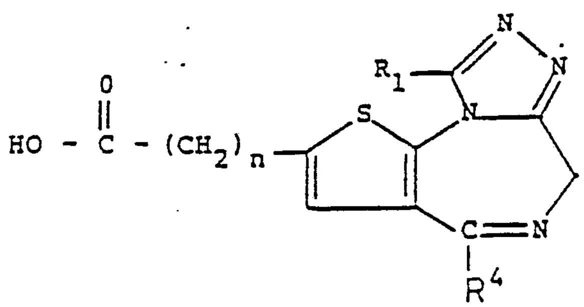

(II)

worin die Reste $R_1$ und $R_4$ die angegebene Bedeutung besitzen, erhalten werden, z. B.

a) durch Umsetzung mit einem entsprechenden Amin in Gegenwart eines Carbodiimids oder Carbonyldiimidazols,

b) durch Überführung der freien Säure in ein Säurehalogenid oder Säureanhydrid und anschließende Umsetzung mit einem entsprechenden Amin.

Die Umsetzung der freien Säure mit dem Amin erfolgt in Gegenwart eines Carbodiimids, beispielsweise von Cyclohexylcarbodiimid, oder Carbonyldiimidazols in einem inerten Lösungsmittel wie Dimethylformamid, Tetrahydrofuran oder Dioxan bei Temperaturen zwischen 0 °C und dem Siedepunkt des Reaktionsgemisches.

Bei der Reaktion des Amins mit einem Säurehalogenid oder Säureanhydrid wird das Amin in einem

inerten Lösungsmittel, beispielsweise Dimethylformamid, Tetrahydrofuran, Dioxan oder einem geeigneten Kohlenwasserstoff wie Benzol oder Toluol bei Temperaturen zwischen Raumtemperatur und dem Siedepunkt des Reaktionsgemisches mit dem Säurehalogenid oder dem Säureanhydrid umgesetzt, wobei gegebenenfalls ein säurebindendes Mittel wie Natriumcarbonat, Natriumbicarbonat oder eine tertiäre organische Base, beispielsweise Pyridin oder Triethylamin, zugegeben wird.

Falls es sich bei dem Amin um eine Flüssigkeit handelt, kann die Umsetzung auch in einem Überschuß des Amins ohne zusätzliches Lösungsmittel erfolgen.

Säurehalogenid bzw. Säureanhydrid erhält man aus der freien Säure in üblicher Weise, z. B. durch Reaktion der Säure mit einem Thionylhalogenid bzw. durch Umsetzung eines Alkalisalzes der Säure mit Acetylchlorid oder Chlorameisensäurechlorid.

Nach den vorstehend beschriebenen Verfahren können beispielsweise die folgenden Endprodukte erhalten werden :

2-[4-(2-Chlorphenyl)-9-methyl-6H-thieno[3,2-f][1,2,4]triazolo[4,3-a][1,4]diazepin-2-yl]-ethan-1-carbonsäure-morpholid ;

2-[4-(2-Chlorphenyl)-9-cyclopropyl-6H-thieno[3,2-f][1,2,4]triazolo[4,3-a][1,4]diazepin-2-yl]-ethan-1-carbonsäure-morpholid ;

[4-(2-Chlorphenyl)-9-methyl-6H-thieno[3,2-f][1,2,4]triazolo[4,3-a][1,4]diazepin-2-yl]-carbonsäuremorpholid ;

[4-(2-Chlorphenyl)-9-methyl-6H-thieno[3,2-f][1,2,4]triazolo[4,3-a][1,4]diazepin-2-yl]-carbonsäureamid ;

2-[4-(2-Chlorphenyl)-9-methyl-6H-thieno[3,2-f][1,2,4]triazolo[4,3-a][1,4]diazepin-2-yl]-ethan-1-carbonsäurediethylamid ;

8-[4-(2-Chlorphenyl)-9-cyclopropyl-6H-thieno[3,2-f][1,2,4]triazolo[4,3-a][1,4]diazepin-2-yl]-n-octan-1-carbonsäuremorpholid ;

2-[4-(2-Chlorphenyl)-9-methyl-6H-thieno[3,2-f][1,2,4]triazolo[4,3-a][1,4]diazepin-2-yl]-ethan-1-carbonsäure-N,N-di-(2-hydroxyethyl)amid ;

2-[4-(2-Chlorphenyl)-9-methyl-6H-thieno[3,2-f][1,2,4]triazolo[4,3-a][1,4]diazepin-2-yl]-ethan-1-carbonsäuremethylamid ;

2-[4-(2-Chlorphenyl)-9-methyl-6H-thieno[3,2-f][1,2,4]triazolo[4,3-a][1,4]diazepin-2-yl]-ethan-1-carbonsäureisopropylamid ;

2-[4-(2-Chlorphenyl)-9-methyl-6H-thieno[3,2-f][1,2,4]triazolo[4,3-a][1,4]diazepin-2-yl]-ethan-1-carbonsäuredimethylamid ;

2-[4-(2-Chlorphenyl)-9-methyl-6H-thieno[3,2-f][1,2,4]triazolo[4,3-a][1,4]diazepin-2-yl]-ethan-1-carbonsäure-N'-methyl-piperazid ;

2-[4-(2-Chlorphenyl)-9-methyl-6H-thieno[3,2-f][1,2,4]triazolo[4,3-a][1,4]diazepin-2-yl]-ethan-1-carbonsäurepyrrolidid ;

2-[4-(2-Chlorphenyl)-9-methyl-6H-thieno[3,2-f][1,2,4]triazolo[4,3-a][1,4]diazepin-2-yl]-ethan-1-carbonsäurepiperidid ;

2-[4-(2-Chlorphenyl)-9-cyclopropyl-6H-thieno[3,2-f][1,2,4]triazolo[4,3-a][1,4]diazepin-2-yl]-ethan-1-carbonsäurediethylamid ;

2-[4-(2-Chlorphenyl)-6H-thieno[3,2-f][1,2,4]triazolo[4,3-a][1,4]diazepin-2-yl]-ethan-1-carbonsäuremorpholid ;

2-[4-(2-Chlorphenyl)-9-brom-6H-thieno[3,2-f][1,2,4]triazolo[4,3-a][1,4]diazepin-2-yl]-ethan-1-carbonsäuremorpholid ;

2-[4-(2-Chlorphenyl)-9-methoxy-6H-thieno[3,2-f][1,2,4]triazolo[4,3-a][1,4]diazepin-2-yl]-ethan-1-carbonsäuremorpholid ;

2-[4-Phenyl-9-methyl-6H-thieno[3,2-f][1,2,4]triazolo[4,3-a][1,4]diazepin-2-yl]-ethan-1-carbonsäuremorpholid ;

2-[4-(2-Nitrophenyl)-9-methyl-6H-thieno-[3,2-f][1,2,4]triazolo[4,3-a][1,4]diazepin-2-yl]-ethan-1-carbonsäuremorpholid ;

2-[4-(2-Methylphenyl)-9-methyl-6H-thieno-[3,2-f][1,2,4]triazolo[4,3-a][1,4]diazepin-2-yl]-ethan-1-carbonsäuremorpholid ;

2-[4-(2-Trifluormethylphenyl)-9-methyl-6H-thieno-[3,2-f][1,2,4]triazolo[4,3-a][1,4]diazepin-2-yl]-ethan-1-carbonsäuremorpholid ;

2-[4-(2-Chlorphenyl)-9-methoxy-6H-thieno-[3,2-f][1,2,4]triazolo[4,3-a][1,4]diazepin-2-yl]-ethan-1-carbonsäurediethylamid ;

2-[4-(2-Chlorphenyl)-9-methoxy-6H-thieno-[3,2-f][1,2,4]triazolo[4,3-a][1,4]diazepin-2-yl]-ethan-1-carbonsäurepiperidid ;

2-[4-(2-Chlorphenyl)-9-methoxy-6H-thieno-[3,2-f][1,2,4]triazolo[4,3-a][1,4]diazepin-2-yl]-ethan-1-carbonsäure-N'-methylpiperazid.

Bei den Ausgangsstoffen der allgemeinen Formel II handelt es sich zum großen Teil um neue Verbindungen. Man erhält sie, ausgehend von den entsprechenden Aldehyden, gemäß folgendem Reaktionsschema :

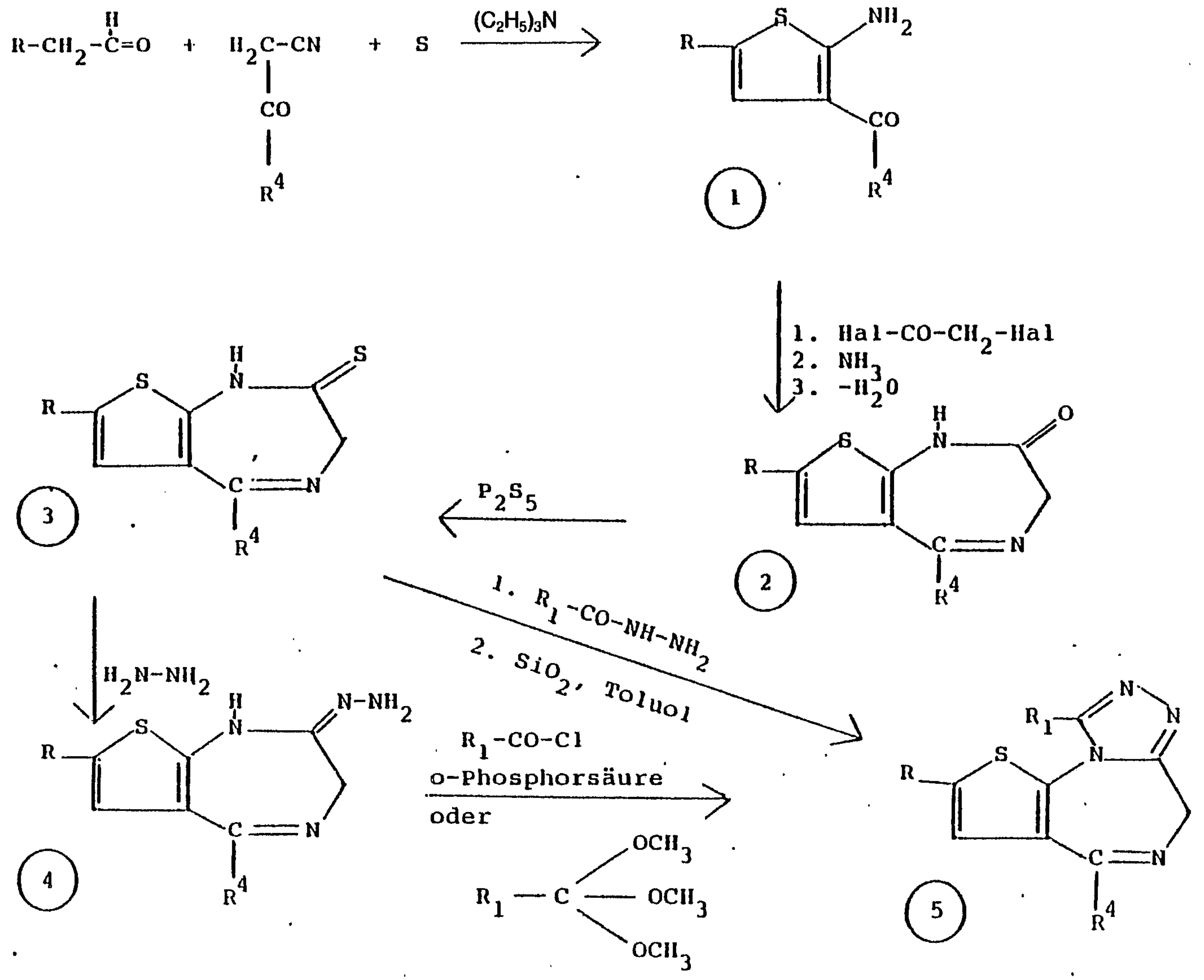


R = Alkylcarbonsäuremethyl- oder -ethylester oder Alkyldicarbonsäuremethyl- oder -ethylester mit 1-8 Kohlenstoffatomen in der Alkylkette.

Falls ein Dicarbonsäureester eingesetzt wurde, wird eine der Carboxylgruppen auf der Stufe des Aminoketons nach dem Verseifen abgespalten.

Für $R_1$ = Wasserstoff geht man entweder von einer Verbindung ③ aus, die mit Ameisensäurehydrazid zu ⑤ umgesetzt wird, oder man läßt auf eine Verbindung ④ einen o-Ameisensäureester einwirken.

Für $R_1$ = Chlor oder Brom wird zunächst eine Verbindung mit $R_1$ = Wasserstoff hergestellt und diese dann mit Chlor oder Brom in Pyridin umgesetzt.

Die 1-Alkoxyverbindung erhält man aus einer oben erwähnten Chlor- oder Bromverbindung durch Umsetzung mit einem Natriumalkoholat.

Verbindungen, in denen n 0 bedeutet, erhält man aus Verbindungen mit einer Carboxylgruppe in 2-Stellung, wie sie in der DE-A-2503235 beschrieben sind ; die freie Säure wird, wie oben ausgeführt, weiter umgesetzt.

Die erfindungsgemäßen Verbindungen besitzen PAF-antagonistische Wirkung.

Bekanntlich handelt es sich bei PAF (Plättchen Aktivierender Faktor) um das Phospholipid Acetylglyceryl-ether-phosphoryl-cholin (AGEPC), das als potenter Lipidmediator bekannt ist, der von tierischen und menschlichen proinflammatorischen Zellen freigesetzt wird. Unter solchen Zellen finden sich hauptsächlich basophile und neutrophile Granulozyten, Makrophagen (aus Blut und Gewebe) sowie Thrombozyten, die an Entzündungsreaktionen beteiligt sind.

PAF zeigt im pharmakologischen Experiment Bronchokonstriktion, Blutdrucksenkung, Auslösung einer Thrombozytenaggregation sowie eine proinflammatorische Wirkung.

Diese experimentell nachweisbaren Wirkungen des PAF weisen direkt oder indirekt auf mögliche Funktionen dieses Mediators in der Anaphylaxie, in der Pathophysiologie des Asthma bronchiale und allgemein bei der Entzündung hin.

PFA-Antagonisten werden benötigt, um einerseits weitere pathophysiologische Funktionen dieses Mediators an Tier und Mensch aufzuklären und andererseits pathologische Zustände und Krankheiten, an denen PAF beteiligt ist, zu behandeln. Beispiele für die Indikationen eines PAF-Antagonisten sind Entzündungsprozesse des Tracheobronchialbaumes (akute und chronische Bronchitis, Asthma bronchiale) oder der Niere (Glomerulonephritis), anaphylaktische Zustände, Allergien und Entzündungen im Bereich der Schleimhäute und der Haut (z. B. Psoriasis) sowie durch Sepsis, Endotoxine oder Verbrennungen bedingte Schockzustände. Weitere wichtige Indikationen für einen PAF-Antagonisten sind Läsionen und Entzündungen im Bereich der Magen- und Darmschleimhaut, wie z. B. Gastritis, im allg. Ulcus pepticum, jedoch insbesondere Ulcus ventriculi und Ulcus duodeni.

Die PAF-antagonistische Wirkung einzelner Benzodiazepine ist bekannt, vgl. E. Kornecki et al., Science 226, 1454-1456 (1984). Diese, als Tranquilizer bzw. Hypnotika ausgewiesenen und im Handel befindlichen Verbindungen sind jedoch wegen ihrer ausgeprägten ZNS-Wirkung zum Einsatz als PAF-Antagonisten in der Therapie in vielen Fällen ungeeignet. Bei den erfindungsgemäßen Verbindungen hingegen fehlt die ZNS-Wirksamkeit, während die PAF-antagonistische Wirkung im Vergleich zu der der bekannten Benzodiazepine um bis zu fünfzigmal größer ist.

Im folgenden werden pharmakologische Untersuchungsergebnisse mitgeteilt :

## Pharmakologische Untersuchungsergebnisse

Die PAF-antagonistische Wirksamkeit einiger Verbindungen der allgemeinen Formel I wurde anhand der Hemmung der Blutplättchen-Aggregation in vitro und der Antagonisierung der durch PAF bewirkten Bronchokonstriktion am narkotisierten Meerschweinchen, Blutdrucksenkung an der narkotisierten Ratte und Hautquaddel an der Ratte untersucht.

Darüber hinaus wurden diese Verbindungen hinsichtlich möglicher Nebenwirkungen auf das zentrale Nervensystem geprüft. Die $LD_{50}$ als Maßstab für die akute Toxizität wurde ebenfalls bestimmt.

### 1. In vitro Untersuchungen : Hemmung der Blutplättchen-Aggregation

Zur Bestimmung der PAF-antagonistischen Wirkung von Substanzen wurde die durch PAF induzierte Aggregation von Humanthrombozyten in vitro verwendet.

Zur Gewinnung von thrombozytenreichem Plasma (TRP) erfolgt die Blutentnahme aus einer nicht gestauten Vene mit Hilfe einer Plastikspritze, in der sich eine 3,8 %ige Natriumcitratlösung befindet. Das Verhältnis zwischen Natriumcitratlösung und Blut beträgt 1 : 9. Nach vorsichtiger Durchmischung wird das Citratblut bei 150 × g (1 200 U/min) 20 Minuten lang zentrifugiert. Die Messung der Thrombozytenaggregation erfolgt nach dem von Born und Cross ausgearbeiteten Verfahren [G. V. R. Born und M. J. Cross, J. Physiol. 168, 178 (1963)], wobei dem TRP unter ständigem Rühren PAF als Auslöser der Aggregation zugesetzt wird.

Die Prüfsubstanz wird jeweils 2-3 Minuten vor Auslösung der Aggregation in einem Volumen von 10 µl zugesetzt. Als Lösungsmittel dienen entweder Aqua. dest., Ethanol und/oder Dimethylsulfoxyd. Kontrollansätze erhalten entsprechende Volumina dieser Lösungsmittel. Nach Registrierung der Ausgangsabsorption (2-3 Minuten) wird die Aggregation mit PAF ($5 \times 10^{-8}$ M) induziert.

Zur Beurteilung von Substanzeffekten wird das Maximum der ersten Aggregationswelle verwendet. Die durch PAF induzierte maximale Absorptionsrate (= maximale Aggregation × 100 %) wird jeweils

gleichzeitig in einem Parallelansatz (= Kontrollansatz in einem der Kanäle des 2 Kanal-Aggregometers) zu jedem Testansatz (zweiter Kanal) mitgeprüft und als 100 %-Wert verwendet.

Der unter dem Einfluß der Testsubstanz erreichte Aggregationswert wird als 100 % — angegeben.

Jede Prüfsubstanz wird bei Konzentrationen von $10^{-3}$ bis $10^{-8}$ M mit einem Stichprobenumfang von jeweils n = 4 hinsichtlich einer hemmenden Wirkung auf die durch PAF induzierte Thrombozytenaggregation untersucht. Danach wird eine Konzentrations-Wirkungskurve anhand von 3 Konzentrationen erstellt und die $IK_{50}$ (Konzentration bei einer 50 %igen Aggregationshemmung) ermittelt. Die IK-Werte von Verbindungen der allgemeinen Formel I bewegen sich zwischen 0,17 und 1,5 µM. Dabei traten 2-[4-(2-Chlorphenyl)-9-methyl-6H-thieno[3,2-f][1,2,4]triazolo[4,3-a][1,4]diazepin-2-yl]-ethan-1-carbonsäurediethylamid und 2-[4-(2-Chlorphenyl)-9-methyl-6H-thieno[3,2-f][1,2,4]triazolo[4,3-a][1,4]diazepino-2-yl]-ethan-1-carbonsäuremorpholid mit $IK_{50}$-Werten von 0,7 bzw. 0,17 als besonders potente Verbindungen hervor.

2. In vivo Untersuchungen

2.1. Antagonisierung der durch PAF bewirkten Bronchokonstriktion an narkotisierten Meerschweinchen

Spontan atmende männliche, 300-450 g schwere Meerschweinchen werden 1 Stunde vor einer i. v. Infusion von PAF (30 ng/(kg × min)) mit der Testsubstanz oder einem Kontrollvehikel oral vorbehandelt. Die Versuchstiere werden dann mit 2 mg/kg Urethan intraperitoneal anästhesiert, worauf die Vena jugularis, die Arteria carotis und die Trachea kanüliert werden. Die PAF-Infusion induziert bei den Kontrolltieren eine starke, anhaltende Bronchokonstriktion, die anhand des Atemzugvolumens, der Compliance und der Resistance gemessen wird und ebenso eine Senkung des Blutdruckes. Nach ca. 7-10 Minuten tritt der Tod ein. Mit den beschriebenen PAF-Antagonisten können diese Effekte auf Atmung und Blutdruck sowie der Eintritt des Todes verhindert werden. Die dafür erforderlichen Dosen bewegen sich zwischen 0,5 und 5 mg/kg p. o. und 0,5 bis 1,0 mg/kg i.v.

2.2. Antagonisierung der durch PAF bewirkten Blutdrucksenkung an der narkotisierten Ratte

Normotone, männliche, 200-250 g schwere Wistar-Ratten werden mit 2 mg/kg Urethan interperitoneal anästhesiert. Die Arteria carotis und Vena jugularis werden kanüliert. Eine intravenöse PAF-Infusion (30 ng/(kg × min)) induziert bei den Kontrolltieren eine starke, anhaltende Blutdrucksenkung. Diese kann dosisabhängig durch intravenöse Injektionen (kumulative Verabreichung) der beschriebenen Verbindungen in einem Dosisbereich von 0,01 bis 0,5 mg/kg aufgehoben werden. Auch eine orale oder intravenöse Verabreichung der Verbindung vor Beginn der PAF-Infusion kann die blutdrucksenkende Wirkung der oben genannten PAF-Infusion dosisabhängig verhindern.

2.3. Antagonisierung der durch PAF induzierten Hautquaddel an der Ratte [Modifiziert nach P. P. Koelzer und K. H. Wehr, Arzneim.-Forsch. 8, 181 (1958)].

Eine intrakutane Injektion von PAF induziert eine Hautquaddel, die Ausdruck einer durch PAF bedingten Erhöhung der Gefäßpermeabilität ist.

Männlichen Wistar-Ratten mit einem Körpergewicht von 250 ± 20 g wird die Bauchdecke (kurz) geschoren. Danach werden 1 ml/kg einer 1 %igen Trypanblau-Lösung durch eine Schwanzvene den Tieren injiziert. Symmetrisch zur Mittellinie (linea alba) werden an drei Stellen in Abständen von ca. 1,5 cm intrakutane Injektionen von physiologischer Kochsalzlösung oder PAF-Lösung (12,5 bis 25,0 ng/Stelle in 0,1 ml) verabreicht. Während an der Injektionsstelle der Kochsalzlösung keine Reaktion auftrat, bewirkte PAF eine Hautreaktion (Quaddel), die durch eine Blaufärbung unterschiedlicher Intensität — abhängig von der PAF-Dosis — sichtbar wurde. Durch gleichzeitige intrakutane Verabreichung der beschriebenen Verbindungen in Dosen von 0,5 bis 5 µg/Stelle (in 0,1 ml) oder durch eine intravenöse Vorbehandlung in Dosen von 0,2 bis 3 mg/kg konnte diese durch PAF induzierte Hautreaktion verhindert werden.

3. Wirkungen auf das zentrale Nervensystem

Es ist allgemein bekannt, daß Substanzen dieses Strukturtyps zentralnervöse Effekte verursachen, die jedoch für eine Verbindung mit PAF-antagonistischer Wirkung nicht erwünscht sind. Daher wurden die beschriebenen Verbindungen hinsichtlich ihrer hypnogenen und antikonvulsiven Wirkung sowie ihres Einflusses auf die Lokomotion geprüft. Eine mögliche hypnotische Wirkung wurde an 400-450 g schweren Meerschweinchen untersucht. Dosen bis zu 200 mg/kg p. o. dieser Substanzen waren nicht in der Lage, eine hypnotische oder sedative Wirkung an diesen Tieren zu erzeugen.

Zur Prüfung einer antikonvulsiven Wirkung kann der Pentetrazol-Antagonismus bei Mäusen (20 bis 25 g Körpergewicht) verwendet werden (M. I. Gluckmann, Current Therapeutic Research, 7 : 721, 1965). Dosen bis zu 100 mg/kg p. o. dieser Verbindungen (1 Stunde vor Pentetrazol) zeigten in diesem Test

keinen Einfluß auf die durch Pentetrazol (125 mg/kg i. p., LD 100) hervorgerufene Mortalität.

Die Wirkung auf die Nachtmotilität (Lokomotion) von Mäusen (20-25 g Körpergewicht) kann in einem Lichtschrankenkäfig untersucht werden. Dabei wird die Anzahl der Lichtstrahlunterbrechungen gemessen. Dosen bis zu 300 mg/kg p. o. der oben genannten Verbindungen zeigten keine Aktivität.

4. Akute Toxizität an Mäusen

Die mittlere letale Dosis ($LD_{50}$) nach oraler Applikation lag für die untersuchten Verbindungen zwischen 3 und 4 g/kg. Die $LD_{50}$-Werte nach intravenöser Verabreichung bewegten sich zwischen 400 und 700 mg/kg.

Die neuen Verbindungen der allgemeinen Formel I können warmblütigen Tieren topisch, oral, parenteral oder durch Inhalation verabreicht werden. Die Verbindungen liegen hierbei als aktive Bestandteile in üblichen Darreichungsformen vor, r. B. in Zusammensetzungen, die im wesentlichen aus einem inerten pharmazeutischen Träger und einer effektiven Dosis des Wirkstoffes bestehen, wie z. B. Tabletten, Dragees, Kapseln, Oblaten, Pulver, Lösungen, Suspensionen, Inhalationsaerosole, Salben, Emulsionen, Sirupe, Suppositorien usw. Eine wirksame Dosis der erfindungsgemäßen Verbindungen liegt bei oraler Anwendung zwischen 1 und 50, vorzugsweise zwischen 3 und 20 mg/Dosis, bei intravenöser oder intramuskulärer Anwendung zwischen 0,01 und 50, vorzugsweise zwischen 0,1 und 10 mg/Dosis. Für die Inhalation sollen Lösungen, die 0,01 bis 1,0, vorzugsweise 0,1 bis 0,5 % Wirkstoff enthalten, eingesetzt werden.

Die folgenden Beispiele dienen zur näheren Erläuterung der Erfindung :

Beispiel 1

2-[4-(2-Chlorphenyl)-9-methyl-6H-thieno[3,2-f][1,2,4]triazolo[4,3-a][1,4]diazepin-2-yl]-ethan-1-carbonsäuremorpholid

5,3 g (0,014 Mol) 2-[4-(2-Chlorphenyl)-9-methyl-6H-thieno[3,2-f][1,2,4]triazolo[4,3-a][1,4]diazepin-2-yl]-ethan-1-carbonsäure, 1,8 g N-Hydroxybenztriazol (HOBT) und 60 ml absolutes Dimethylformamid werden unter Rühren bei Raumtemperatur mit 1,2 g (0,014 Mol) Morpholin versetzt, wobei eine klare Lösung erfolgt. Bei 0-5 °C fügt man anschließend während 5 bis 10 Minuten 3,5 g Dicyclohexylcarbodiimid in fester Form hinzu und hält die Temperatur weitere 6-8 Stunden bei 0-10 °C. Der ausgefallene Dicyclohexylharnstoff wird abgesaugt, mit wenig kaltem Dimethylformamid nachgewaschen und das Filtrat im Vakuum eingedampft. Man löst den Rückstand in Methylenchlorid, wäscht mit 5 %iger Sodalösung und Eiswasser, dampft die organische Phase ein und bringt den Rückstand mit Essigester zur Kristallisation. Ausbeute : 5,2 g (83,2 % d. Th.) farblose Kristalle, Fp. 189-190 °C.

$^1$H-NMR ($CDCl_3$), $\delta = 2,64$ (2t, $-CH_2-CO-$), 2,71 (3s, $CH_3$), 3,17 (2t, $CH_2$), 3,33-3,81 (8m, Morpholin), 4,96 (2s, $CH_2$), 6,48 (1s, Thiophen), 7,28-7,60 (4m, Aryl).

Das Ausgangsmaterial wird wie folgt erhalten ; es ist neu :

a) 2-Amino-3-o-chlorbenzoyl-5-(2,2-dicarbethoxyethyl)-thiophen

53,9 g (0,3 Mol) o-Chlorcyanoacetophenon, 9,6 g Schwefel, 30,4 g (0,3 Mol) Triethylamin und 120 ml Dimethylformamid werden unter Rühren, beginnend bei Raumtemperatur mit 64,8 g (0,3 Mol) Dicarbethoxybutyraldehyd [D. T. Warner, J. Am. Chem. Soc. 70, 3470 (1948) ; Kp. 97 °C/0,1 mBar] versetzt, wobei die Temperatur auf 45-50 °C ansteigt. Man rührt 2-3 Stunden bei 60-70 °C, kühlt auf Raumtemperatur und fügt 400 ml Wasser hinzu. Das gebildete Thiophenderivat wird dreimal mit jeweils 200 ml Methyl-tert.-butylketon ausgeschüttelt. Nach dem Waschen mit Wasser und Trocknen der organischen Phase wird eingedampft und der kristalline Rückstand aus Isopropanol/Wasser 7 : 3 umkristallisiert.

Ausbeute : 90 g (74 % d. Th.), Fp. 96-98 °C.

b) 2-Amino-o-chlorbenzoyl-5-(2-carbomethoxyethyl)-thiophen

63 g (0,15 Mol) obiger Verbindung werden mit 120 ml Ethanol und 32,5 g Ätzkali in 50 ml Wasser 2 Stunden unter Rückfluß gekocht. Man dampft im Vakuum ein, verdünnt mit 50 ml Wasser und säuert mit HCl an. Die schmierig ausgefallene Säure wird mehrmals mit Essigester ausgeschüttelt. Die Extrakte werden getrocknet und eingedampft, der Rückstand mit 300 ml Toluol und 30 ml Dimethylformamid 2 Stunden unter Rückfluß gekocht. Nach dem Eindampfen auf ca. 50 ml erhält man Kristalle der Monocarbonsäure.

Ausbeute : 20,5 g. Die gereinigte Säure schmilzt bei 171-173 °C.

Die Rohsäure wird zusammen mit 400 ml absolutem Methanol und 0,4 ml konzentrierter Schwefelsäure 18 Stunden bei Raumtemperatur gerührt. Man gießt nach Abdampfen des Methanols auf Eis, schüttelt mit Methylenchlorid aus und erhält nach erneutem Eindampfen aus Isopropylether 15 g Ester vom Fp. 89-90 °C.

c) 2-Bromacetylamino-3-o-chlorbenzoyl-5-(2-carbomethoxyethyl)-thiophen

27,8 g (0,09 Mol) obigen Esters werden in 700 ml Toluol suspendiert und mit 10 g Natriumbicarbonat in 57 ml Wasser versetzt. Unter Rühren fügt man allmählich bei 40-50 °C 7,9 ml Bromacetylbromid hinzu und rührt 30 Minuten nach. Man wäscht mit wasser, trocknet die Toluolphase, dampft im Vakuum ein und bringt mit Isopropylether zur Kristallisation.

Ausbeute : 35-37 g, Fp. 104-106 °C.

d) 2-Aminoacetylamino-3-o-chlorbenzoyl-5-(2-carbomethoxyethyl)-thiophen

35,8 g (0,08 Mol) obiger Bromacetylverbindung werden in 700 ml Essigester gelöst und unter Rühren 2-3 Stunden bei Raumtemperatur trockener Ammoniak eingeleitet. Man läßt über Nacht stehen, wäscht mit Eiswasser, trocknet, dampft ein und erhält 22-25 g ölige Aminoverbindung.

e) 7-(2-Carbomethoxyethyl)-5-o-chlorphenyl-thieno-1,4-diazepinon

21,3 g (0,056 Mol) obiger Verbindung werden in 500 ml Toluol gelöst und mit 75 g Kieselgel am Wasserabscheider 2 Stunden unter Rückfluß gekocht. Man saugt das $SiO_2$ ab und extrahiert das Diazepin mit heißem Methanol. Nach Eindampfen des Methanols werden 12-15 g Diazepin vom Fp. 160-162 °C erhalten.

f) 7-(2-Carbomethoxyethyl)-5-o-chlorphenyl-thieno-1,4-diazepin-2-thion

10 g (0,03 Mol) obigen Diazepinons werden in 100 ml Diglyme mit 6,8 g Phosphorpentasulfid und 5 g Natriumhydrogencarbonat 3 Stunden bei 70-80 °C gerührt. Man gießt die Suspension auf Eis, rührt 30-45 Minuten und saugt die Kristalle ab. Nach dem Trocknen erhält man 10 g Thion vom Fp. 185-186 °C.

g) 2-[4-(2-Chlorphenyl)-9-methyl-6H-thieno[3,2-f][1,2,4]triazolo[4,3-a][1,4]diazepin-2-yl]-ethan-1-carbonsäuremethylester

6,1 g (0,016 Mol) obiger Schwefelverbindung werden in 100 ml Tetrahydrofuran gelöst und nach Zugabe von 1 g Hydrazinhydrat 30 Minuten bei 45-50 °C gerührt. Anschließend wird im Vakuum eingedampft. Es hinterbleiben 5-5,2 g Öl, die aus Isopropylether kristallisieren (Fp. 175-177 °C).

Die Hydrazinoverbindung ergibt beim Erhitzen in 35 ml Ortho-Essigsäureester auf 80 °C, Eindampfen aus Methylenchlorid-Ether 3 g des Triazolodiazepins vom Fp. 114-115 °C.

Dieselbe Verbindung ist aus dem Thion mit Essigsäurehydrazid zugänglich.

Aus 6,1 g des Methylesters erhält man nach Verseifen in alkoholisch-wäßriger Kalilauge 5,7-5,8 g der freien Carbonsäure vom Fp. 196-198 °C.

## Beispiel 2

2-[4-(2-Chlorphenyl)-9-cyclopropyl-6H-thieno[3,2-f][1,2,4]triazolo[4,3-a][1,4]diazepin-2-yl]-ethan-1-carbonsäuremorpholid

Ausgehend von 15 g 2-[4-(2-Chlorphenyl)-9-cyclopropyl-6H-thieno[3,2-f][1,2,4]triazolo[4,3-a][1,4]diazepin-2-y]-ethan-1-carbonsäure, Fp. 227-230 °C, wird mit Hilfe von Dicyclohexylcarbodiimid und Morpholin nach dem im Beispiel 1 beschriebenen Verfahren die Titelverbindung erhalten.

Ausbeute : 15,0 g (86,5 % d. Th.), Fp. 159-160 °C.

$^1$H-NMR ($CDCl_3$) ; $\delta$ = 0,96-1,40 (4m, Cyclopropyl —$CH_2$—), 1,93-2,28 (1m, Cyclopropyl) 2,64 (2t, $CH_2$—CO—), 3,15 (2t, $CH_2$), 3,31-3,77 (8m, Morpholin), 4,91 (2s, $CH_2$-7-Ring) 6,44 (1s, Thiophen), 7,22-7,60 (4m, Aryl).

Die Ausgangsverbindung erhält man wie folgt :

38 g (0,1 Mol) 7-(2-Carbomethoxyethyl)-5-o-chlorphenyl-thieno-1,4-diazepin-2-thion (vgl. Beispiel 1) vom Fp. 185-186 °C werden mit 11 g Cyclopropylcarbonsäurehydrazid in 50 ml Dioxan 1 Stunde unter Rückfluß gekocht. Nach dem Eindampfen hinterbleiben beim Verreiben mit Ether 30 g rote Kristalle vom Fp. 148-150 °C, die man in 1 l Toluol in Gegenwart von 140 g Kieselgel 4 Stunden am Wasserabscheider erhitzt. Anschließend wird abgekühlt, abgesaugt und die Triazoloverbindung mit heißem Methanol extrahiert. Es verbleiben als Rückstand der Extraktion 24 g zähes Öl, das man durch einstündiges Kochen in 250 ml 2 n alkoholischer Kaligauge verseift. Nach üblicher Aufarbeitung werden 15-18 g der Carbonsäure erhalten, die direkt in das Amid überführt werden kann.

## Beispiel 3

[4-(2-Chlorphenyl)-9-methyl-6H-thieno[3,2-f][1,2,4]triazolo[4,3-a][1,4]diazepin-2-yl]-carbonsäuremorpholid

36 g (0,1 Mol) der entsprechenden Carbonsäure (K. H. Weber et al. DE-A-2503235, 29.7.1976, S. 14) vom Fp. 302 °C werden, wie in Beispiel 1 beschrieben, mit 10 g Morpholin in Gegenwart von Dicyclohexylcarbodiimid in Dimethylformamid umgesetzt. Man erhält 38 g (89 % d. Th.) zähes Öl.

$^1$H-NMR ($CDCl_3$) ; δ = 2,75 (3s, $CH_3$), 3,71 (8s, Morpholin), 4,97 (2s $CH_2$-7-Ring) 6,83 (1s, Thiophen) 7,26-7,64 (4m, Aryl).

Ausgehend von 2-Amino-3-(2-chlorbenzoyl)-thiophen-5-carbonsäure [O. Hromatka, Monatsh. Chem. 164, 973 (1973)] erhält man über das entsprechende Säurechlorid das Morpholid vom Fp. 206-208 °C. Dieses läßt sich auf dem in Beispiel 1 beschriebenen Weg ebenfalls in die Titelverbindung überführen.

## Beispiel 4

[4-(2-Chlorphenyl)-9-methyl-6H-thieno[3,2-f][1,2,4]triazolo[4,3-a][1,4]diazepin-2-yl]-carbonsäureamid

3,7 g (0,01 Mol) des entsprechenden Carbonsäuremethylesters (K. H. Weber et al., DE-A-2503235, 29.7.1976, S. 14), vom Fp. 230-232 °C, werden in 100 ml Methanol gelöst und unter Rühren bei Raumtemperatur Ammoniak bis zur Sättigung eingeleitet. Anschließend rührt man 2 Tage bei 20-25 °C weiter, dampft das Lösungsmittel ab und chromatographiert über $SiO_2$ (Elution mit Methylenchlorid/Methanol 9 : 1).

Ausbeute : 3,5 g (98 % d. Th.), Fp. 300 °C (Zers.).

$^1$H-NMR ($CDCl_3$) ; δ = 2,66 (3s, $CH_3$), 4,85 (2s, $CH_2$), 7,50 (1s, Thiophen), 7,52 (4H, Aryl), 7,65 und 8,25 (2s, $NH_2$).

## Beispiel 5

2-[4-(2-Chlorphenyl)-9-methyl-6H-thieno[3,2-f][1,2,4]triazolo[4,3-a][1,4]diazepin-2-yl]-ethan-1-carbonsäurediethylamid

3,87 g (0,01 Mol) der entsprechenden Carbonsäure (siehe Beispiel 1) werden in 50 ml Methylenchlorid mit 1 ml reinem Thionylchlorid 2 Stunden bei 30-35 °C gerührt. Anschließend fügt man unter Eiskühlung 8 ml Diethylamin hinzu und rührt 30 Minuten nach. Die Salze werden mit Wasser ausgewaschen, die Methylenchloridphase getrocknet, teilweise eingedampft und zur Endreinigung über $SiO_2$ chromatographiert (Laufmittel Methylenchlorid mit 4 % Methanolzusatz). Man erhält 1,8-2,0 g zähes Öl.

$^1$H-NMR ($CDCl_3$) ; δ = 1,09 und 1,12 (2 × 3 t, $C_2H_5$), 2,52 (2t, $CH_2$—CO), 2,72 (3s, $CH_3$), 3,03-3,56 (6m, $C_2H_5$ und $CH_2$), 4,90 (2s, $CH_2$-7-Ring), 6,44 (1s, Thiophen), 7,25-7,55 (4m Aryl).

## Beispiel 6

[4-(2-Chlorphenyl)-9-methyl-6-H-thieno[3,2-f][1,2,4]triazolo[4,3-a][1,4]diazepin-2-yl]-methancarbonsäure-morpholid

20 g (0,054 Mol) [4-(2-Chlorphenyl)-9-methyl-6H-thieno[3,2-f][1,2,4]triazolo[4,3-a][1,4]diazepin-2-yl]-methancarbonsäure, 500 ml Tetrahydrofuran und 10 g 1,1'-Carbonyldiimidazol werden 1 Stunde bei Raumtemperatur gerührt und die klar gewordene Lösung mit 0,06 Mol (5,2 g) Morpholin versetzt. Nach Rühren über Nacht bei Raumtemperatur, Eindampfen, Aufnehmen in Methylenchlorid, Waschen mit Natriumbicarbonat-Lösung und Filtration über eine $SiO_2$-Säule, konnten durch Umkristalisation aus Essigester 9,4 g der Titelverbindung vom Fp. 143-144 °C erhalten werden.

$^1$H-NMR ($CDCl_3$) : δ = 2,74 (3s, $CH_3$), 3,36-3,82 (8m, Morpholin), 3,88 (2s $CH_2$—CO), 4,97 (2s, $CH_2$-7-Ring), 6,51 (1s, Thiophen), 7,25-7,60 (4m, Aryl).

Die Carbonsäure ist auf folgendem Weg zugänglich :

Malonsäurediethylester und Bromacetaldehydacetal ergeben nach literaturbekannten Verfahren den Dicarbethoxypropionaldehyd ($Kp_{0,01}$ : 92-95 °C), der sich analog Beispiel 1 mit Chlorcyanoacetophenon und Schwefel in das entsprechende 2-Aminobenzoylthiophen umwandeln läßt. Verseifung, Decarboxylierung und Veresterung mit Methanol/Schwefelsäure ergeben das 2-Amino-3-(o-chlorbenzoyl)-5-(carbomethoxy-methyl)-thiophen, Bromacetylierung, Aminierung und Ringschluß führten zu dem entsprechenden Diazepinon vom Fp. 180-182 °C. Das hieraus erhaltene Thion schmilzt bei 184-185 °C. Behandlung des Thions mit Hydrazin und nachfolgende Umsetzung mit Orthoessigsäureester führt zum Triazolothienocarbonsäuremethylester vom Fp. 139-141 °C ; anschließende Verseifung mit alkoholisch-wässeriger Kalilauge ergibt die freie Carbonsäure vom Fp. 257-259 °C.

## Beispiel 7

3-[4-(2-Chlorphenyl)-9-methyl-6-H-thieno[3,2-f][1,2,4]triazolo[4,3-a][1,4]diazepin-2-yl]-propan-1-carbonsäuremorpholid

Analog Beispiel 6 werden 10 g (0,025 Mol) 3-[4-(2-Chlorphenyl)-9-methyl-6-H-thieno[3,2-f][1,2,4]triazolo[4,3-a][1,4]diazepin-2-yl]-propan-1-carbonsäure in Tetrahydrofuran mit Morpholin und 1,1'-Carbonyldiimidazol umgesetzt und ergeben nach der chromatographischen Aufarbeitung 10,5 g (89 % d. Th.) eines zähen fast farblosen Öls.

$^1$H-NMR ($CDCl_3$) : δ 2,00 (2m, $CH_2$), 2,37 (2m, $CH_2CO$), 2,71 (3s, $CH_3$), 2,87 (2m, $CH_2$), 3,26-3,83 (8m, Morpholin), 4,94 (2s, $CH_2$-7-Ring), 6,41 (1s, Thiophen), 7,24-7,61 (4m, Aryl).

Das Ausgangsmaterial erhielten wir auf folgendem Weg :

Ausgehend von Cyclohexanon ist nach literaturbekannter Methode (L. Claisen, Ber. dtsch. chem. Ges. 40, 3907) der Enolether zugänglich, den man einer Ozonolyse [V. Schmid, P. Grafen, Liebigs Ann. Chem. 656, 97 (1962)] unterwirft. Erhalten wird der 5-Formylvaleriansäuremethylester, der wie bereits beschrieben zum 2-Amino-benzoyl-thiophen-2-propancarbonsäureester umgesetzt wird. Das hieraus erhaltene Thiophendiazepinon schmilzt bei 152-153 °C. Die weitere Umsetzung mit Phosphorpentasulfid analog Beispiel 1 f ergibt das Thion vom Fp. 176-178 °C. Die Umsetzung mit Hydrazin und Reaktion mit Orthoessigsäureester nach Beispiel 1 g führen zu dem entsprechenden Ester, der mit alkoholischer Kalilauge zur Carbonsäure vom Schmelzpunkt 257-259 °C verseift wird.

## Beispiel 8

4-[4-(2-Chlorphenyl)-9-methyl-6-H-thieno[3,2-f][1,2,4]triazolo[4,3-a][1,4]diazepin-2-yl]-butan-1-carbonsäuremorpholid

15 g (0.036 Mol) 4-[4-(2-Chlorphenyl)-9-methyl-6H-thieno[3,2-f][1,2,4]triazolo[4,3-a][1,4]diazepin-2-yl]-butan-1-carbonsäure ergeben analog Beispiel 1 mit Morpholin und Dicyclohexylcarbodiimid in Dimethylformamid als Lösungsmittel die Titelverbindung als hellgelbes zähes Öl (Ausbeute 13 g = 75 % d. Th.).

$^1$H-NMR ($CDCl_3$) ; δ 1,72 (4m, $-CH_2-CH_2-$), 2,87 (2m, $CH_2CO$), 2,71 (3s, $CH_3$), 2,83 (2m, $CH_2$), 3,30-3,77 (8m, Morpholin), 4,93 (2s, $CH_2$-7-Ring), 6,41 (1s, Thiophen), 7,21-7,58 (4m, Aryl).

Die Carbonsäure wird ausgehend von käuflichem Cycloheptanon analog Beispiel 7 über den entsprechenden Aldehyd vom $Kp_{15}$ : 115-120 °C, dem Thienotriazolo-1,4-diazepin-2-butancarbonsäuremethylester vom Fp. 119-121 °C nach Verseifung desselben erhalten und schmilzt bei 133-134 °C.

## Beispiel 9

2-[9-Brom-4-(2-chlorphenyl-6-H-thieno[3,2-f][1,2,4]triazolo[4,3-a][1,4]diazepin-2-yl]-ethan-1-carbonsäuremorpholid

4,4 g (0,001 Mol) 2-[4-(2-Chlorphenyl)-6-H-thieno[3,2-f][1,2,4]triazolo[4,3-a][1,4]diazepin-2-yl]-ethan-1-carbonsäuremorpholid vom Fp. 188-189 °C (vgl. Beispiel 19) wird in 44 ml Chloroform gelöst, 2 ml Pyridin und 0.7 ml Brom zugefügt und über Nacht bei Raumtemperatur gerührt. Die hellbraune Reaktionslösung wird mit Natriumbicarbonat/Wasser gewaschen, die organische Phase getrocknet und das Lösungsmittel eingeengt, bei Zusatz von Ether werden 3,1 g (60 % d. Th.) der Titelverbindung als helle braungraue Kristalle erhalten, die bei 181-182 °C schmelzen.

$^1$H-NMR ($CDCl_3$) : δ = 2,66 (2t, $CH_2CO$), 3,17 (2t, $CH_2$), 3,31-3,75 (8m, Morpholin), 4,95 (2s, $CH_2$), 6,46 (1s, Thiophen), 7,30-7,55 (4m, Aryl).

## Beispiel 10

2-[4-(2-Chlorphenyl)-9-methoxy-6-H-thieno[3,2-f][1,2,4]triazolo[4,3-a][1,4]diazepin-2-yl]-ethan-1-carbonsäuremorpholid

2,6 g (0,5 mMol) der 9-Bromverbindung von Beispiel 9 werden mit einer Lösung von 3,7 g Ätzkali in 400 ml Methanol 1 Stunde bei 50-60 °C gerührt. Nach Abdampfen des Methanols wird der Rückstand mit Eiswasser versetzt und mit Methylenchlorid extrahiert. Die organische Phase wird getrocknet und das Lösungsmittel im Vakuum eingeengt. Der verbleibende Rückstand wird aus Essigester umkristallisiert, wobei man 1,8 g (76 % d. Th.) der Titelverbindung vom Fp. 163-164 °C erhält.

$^1$H-NMR ($CDCl_3$) : δ = 2.62 (2t, $CH_2$), 3,11 (2t, $CH_2$), 3,32-3,77 (8m, Morpholino), 4,27 (3s, $CH_3$), 4,88 (2s, $CH_2$), 6,36 (1s, Thiophen), 7,36 (4s, Aryl).

## Beispiel 11

8-[4-(2-Chlorphenyl)-9-cyclopropyl-6H-thieno[3,2-f][1,2,4]triazolo[4,3-a][1,4]diazepin-2-yl]-n-octan-1-carbonsäuremorpholid

4,63 g (0,01 Mol) der entsprechenden Carbonsäure werden analog dem in Beispiel 1 beschriebenen Verfahren mittels Cyclohexylcarbodiimid in das Amid überführt.

Man erhält 4,4 g (84,6 % d. Th.) zähes Öl.

$^1$H-NMR ($CDCl_3$) ; δ = 1,12-1,14 (12m, $(CH_2)_6$), 2,72 (3s, $CH_3$), 2,68 (2t, $CH_2-CO$), 3,15 (2t, $CH_2$), 3,31-3,78 (8m, Morpholin), 4,90 (2s, $CH_2$), 6,42 (1s, Thiophen), 7,25-7,60 (4m, Aryl).

Ausgangsverbindung für die Carbonsäure ist der 9-Formylnonancarbonsäuremethylester vom $Kp_{005}$ 101-104 °C, den man nach R. A. Volkmann et al., J. Org. Chem. 48, 1767 (1983) erhält.

Nach den vorstehend beschriebenen Methoden wurden ferner die folgenden Verbindungen erhalten :

| Beispiel Nr. | $R_1$ | 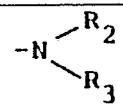 | $R_4$ | n | Fp. °C | Spektrum |
|---|---|---|---|---|---|---|
| 12 | $CH_3$ | 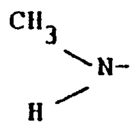 | 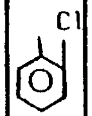 | 2 | 166-167 | $^1$H-NMR ($CDCl_3$); δ = 2,50 (2t, $CH_2$-CO), 2,70 (3s, $CH_3$), 2,79 (3d, $CH_3N$), 3,31 (2t, $CH_2$), 4,85 (2s, $CH_2$), 5,85 (1q, NH), 6,45 (1s, Thiophen), 7,25 - 7,57 (4m, Aryl) |
| 13 | $CH_3$ | 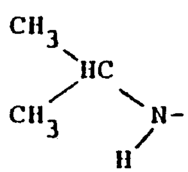 | 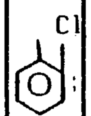 | 2 | Öl | $^1$H-NMR ($CDCl_3$); δ = 1,11 (6d ($CH_3$)-Isoprop.), 2,45 (2t, $CH_2CO$), 2,68 (3s, $CH_3$), 3,12 (2t, $CH_2$), 4,08 (1m CH-Isoprop.), 4,91 (2s, $CH_2$), 5,48 (1d, NHCO), 6,45 (1s, Thiophen), 7,22 - 7,57 (4m, Aryl) |
| 14 | $CH_3$ | 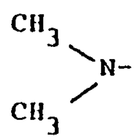 | 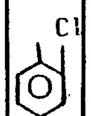 | 2 | 157-158 | $^1$H-NMR ($CDCl_3$); δ = 2,63 (2t, $CH_2$-CO), 2,71 (3s, $CH_3$), 2,97 (6s, $N(CH_3)_2$), 3,13 (2t, $CH_2$), 4,14 (2s, $CH_2$), 6,44 (1s, Thiophen), 7,28 - 7,57 (4m, Aryl) |

| Beispiel Nr. | $R_1$ | 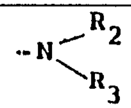 | $R_4$ | n | Fp. °C | Spektrum |
|---|---|---|---|---|---|---|
| 15 | $CH_3$ | 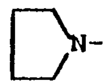 | 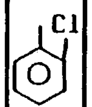 | 2 | 167-168 | $^1$H-NMR ($CDCl_3$); $\delta$ = 1,64 - 2,20 (4m, Pyrrazol), 2,57 (2t, $CH_2$-CO), 2,70 (3s, $CH_3$), 3,14 (2t, $CH_2$), 3,24 - 3,57 (4m, Pyrrazol), 4,41 (2s, $CH_2$), 6,42 (1s, Thiophen), 7,26 - 7,52 (4m, Aryl) |
| 16 | $CH_3$ | 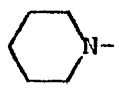 | 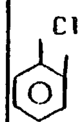 | 2 | 143-145 | $^1$H-NMR ($CDCl_3$); $\delta$ = 1,33 - 1,77 (6m, Piperidin), 2,62 (2t, $CH_2$-CO), 2,71 (3s, $CH_3$), 3,13 (2t, $CH_2$), 3,35, 3,55 (4m, Piperidin), 4,93 (2s, $CH_2$), 6,42 (1s, Thiophen), 7,24 - 7,57 (4m, Aryl) |
| 17 | $CH_3$ | 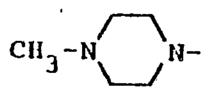 | 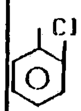 | 2 | Öl | $^1$H-NMR ($CDCl_3$); $\delta$ = 2,32 (3s, $NCH_3$), 2,39 (4t, Piperazin), 2,65 (2t, $CH_2$-CO), 2,73 (3s, $CH_3$), 3,16, 3,46 (4t, Piperazin), 3,65 (2t, $CH_2$), 4,95 (2s, $CH_2$), 6,45 (1s, Thiophen), 7,29 - 7,58 (4m, Aryl) |

| Beispiel Nr. | $R_1$ | 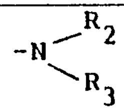 | $R_4$ | n | Fp. °C | Spektrum |
|---|---|---|---|---|---|---|
| 18 | 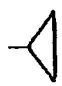 | 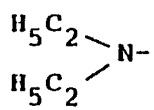 | 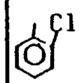 | 2 | 81 | $^1H$-NMR ($CDCl_3$); δ = 0,93 - 1,35 (10m, Cyclopropyl, $C_2H_5$), 1,93 - 2,26 (1m, CH-Cyclopropyl), 2,62 (2t, $CH_2CO$), 3,00 - 3,53 (6m, $C_2H_5$ und $CH_2$), 4,91 (2s, $CH_2$), 6,46 (1s, Thiophen), 7,24 - 7,57 (4m, Aryl) |
| 19 | H | 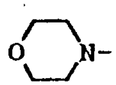 | 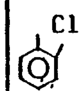 | 2 | 188-190 | $^1H$-NMR ($CDCl_3$); δ = 2,63 (2t, $CH_2$-CO), 3,14 (2t, $CH_2$), 3,32 - 3,67 (8m, Morpholin), 5,03 (2s, $CH_2$), 6,42 (1s, Thiophen), 7,25 - 7,42 (4m, Aryl), 8,46 (1s, Triazol) |
| 20 | $CH_3$ | $NH_2$ | 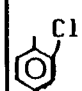 | 1 | 202-204 | $^1H$-NMR ($CDCl_3$); δ = 2,70 (3s, $CH_3$), 3,75 (2s, $CH_2$-$NH_2$), 4,90 (2s, $CH_2$-7-Ring), 5,54 - 6,48 (2m, $NH_2$), 6,53 (1s, Thiophen), 7,18 - 7,56 (4m, Aryl). |

| Beispiel Nr. | $R_1$ | 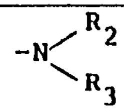 | $R_4$ | n | Fp. °C | Spektrum |
|---|---|---|---|---|---|---|
| 21 | $CH_3$ | 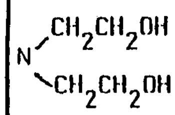 | 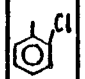 | 2 | Öl | $^1$H-NMR ($CDCl_3$); δ = 2,16 (2s, OH), 2,69 (3s, $CH_3$), 2,79 (2t, $CH_2CO$), 3,15 (2t, $CH_2$), 3,53 - 377 (8m, <u>$CH_2$</u>-<u>$CH_2$</u>-OH, 4,90 (2s, $CH_2$-7-Ring), 6,45 (1s, Thiophen), 7,22 - 7,63 (4m, Aryl). |
| 22 | $CH_3$ | 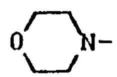 | 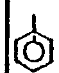 | 2 | 180-181 | $^1$H-NMR ($CDCl_3$); δ = 2,70 (2t, $CH_2$), 2,70 (3s, $CH_3$), 3,22 (2t, $CH_2$), 3,36 - 3,80 (8m, Morpholin), 4,85 (2s, $CH_2$), 6,73 (1s, Thiophen), 7,24 - 7,72 (5m, Aryl) |
| 23 | $CH_3$ | 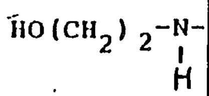 | 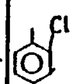 | 2 | Öl | $^1$H-NMR ($CDCl_3$); δ = 3,50 (1s, OH), 2,54 (2t, $CH_2CO$), 2,65 (3s, $CH_3$), 3,11 (2t, $CH_2$), 3,38 (2m, $NCH_2$), 3,65 (2t, $OCH_2$), 4,86 (2s, $CH_2$), 6,44 (1s, Thiophen), 6,88 (1t, NH-CO), 7,25 - 7,55 (4m, Aryl) |

| Beispiel Nr. | $R_1$ | 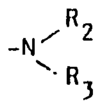 | $R_4$ | n | Fp. °C | Spektrum |
|---|---|---|---|---|---|---|
| 24 | $CH_2Cl$ | 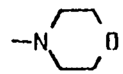 | 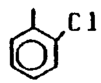 | 2 | 163-165 | $^1$H-NMR ($CDCl_3$): $\delta$ = 2,64 (2t, $CH_2CO$), 3,16 (2t, $CH_2$), 3,28 - 3,78 (8m,Morpholin), 4,94 (4s, $CH_2Cl$ u $CH_2$-7-Ring) 6,41 (1s, Thiophen), 7,17 - 7,53 (4m, Aryl). |
| 25 | $CH_2Br$ | 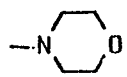 | 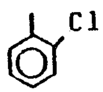 | 2 | 81 | $^1$H-NMR ($CDCl_3$): $\delta$ = 2,58 (2t, $CH_2CO$), 3,11 (2t, $CH_2$), 3,22 - 3,74 (8m, Morpholin), (2s, $CH_2Br$), 4,98 (2s, $CH_2$-7-Ring), (1s, Thiophen), 7,21 - 7,71 (4m, Aryl) |
| 26 | $CH_3-CH_2-CH_2-O$ | 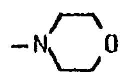 | 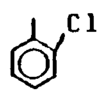 | | | $^1$H-NMR ($CDCl_3$): $\delta$ = 1.09 (3t, J=7Hz, $CH_3$), 1.95 (2m, $CH_2-CH_3$), 2.63 (2t, J=7Hz, $CH_2CO$), 3.15 (2t, J=7Hz, $CH_2$-Thiophen), 3.31-3.74 (8m, Morpholin), 4.60 (2t, J=6Hz, O-$CH_2$), 4.90 (2s; $CH_2$-7-Ring), 6.39 (1s, Thiophen), 7.39 (4s, Aryl). |

Nachfolgend werden Beispiele für einige pharmazeutische Zusammensetzungen unter Verwendung von Verbindungen der allgemeinen Formel I als aktiver Bestandteil angegeben. Falls nicht ausdrücklich anders bezeichnet, handelt es sich bei den Teilen um Gewichtsteile.

1. Tabletten

Die Tablette enthält folgende Bestandteile :

| | | |
|---|---|---|
| Wirkstoff gemäß allgemeines Formel I | | 0,020 Teile |
| Stearinsäure | | 0,010 Teile |
| Dextrose | | 1,890 Teile |
| | gesamt | 1,920 Teile |

Herstellung

Die Stoffe werden in bekannter Weise zusammengemischt und die Mischung zu Tabletten verpreßt, von denen jede 1,92 g wiegt und 20 mg Wirkstoff enthält.

2. Salbe

Die Salbe setzt sich aus folgenden Bestandteilen zusammen :

| | | |
|---|---|---|
| 2-[4-(2-Chlorphenyl)-9-methyl-6H-thieno[3,2-f][1,2,4]triazolo[4,3-a][1,4]diazepin-2-yl]-ethan-1-carbonsäuremorpholid | | 50 mg |
| Neribas Salbe (Handelsware Scherax) | ad | 10 g |

Herstellung

Der Wirkstoff wird mit 0,5 g Salbengrundlage verrieben und die restliche Grundlage in Teilmengen zu 1,0 g nach und nach innig zu einer Salbe vermischt. Man erhält eine 0,5 %ige Salbe. Die Verteilung des Wirkstoffes in der Grundlage wird optisch unter dem Mikroskop kontrolliert.

3. Creme

Zusammensetzung :

| | | |
|---|---|---|
| 2-[4-(2-Chlorphenyl)-9-methyl-6H-thieno[3,2-f][1,2,4]triazolo[4,3-a][1,4]diazepin-2-yl]-ethan-1-carbonsäuremorpholid | | 50 mg |
| Neribas Salbe (Handelsware Scherax) | ad | 10 g |

Herstellung

Der Wirkstoff wird mit 0,5 g Cremegrundlage verrieben und die restliche Grundlage in Teilmengen zu 1,0 g nach und nach mit Pistill eingearbeitet. Man erhält eine 0,5 %ige Creme. Die Verteilung des Wirkstoffes in der Grundlage wird optisch unter dem Mikroskop kontrolliert.

4. Ampullenlösung

Zusammensetzung :

| | | |
|---|---|---|
| a) 2-[4-(2-Chlorphenyl)-9-methyl-6H-thieno[3,2-f][1,2,4]triazolo[4,3-a][1,4]diazepin-2-yl]-ethan-1-carbonsäuremorpholid | | 1,0 mg |
| Natriumchlorid | | 45,0 mg |
| Aqua pro inj. | ad | 5,0 ml |
| b) 2-[4-(2-Chlorphenyl)-9-methyl-6H-thieno[3,2-f][1,2,4]triazolo[4,3-a][1,4]diazepin-2-yl]-ethan-1-carbonsäuremorpholid | | 5,0 mg |
| Natriumchlorid | | 45,0 mg |
| Aqua pro inj. | ad | 5,0 ml |
| c) 2-[4-(2-Chlorphenyl)-9-methyl-6H-thieno[3,2-f][1,2,4]triazolo[4,3-a][1,4]diazepin-2-yl]-ethan-1-carbonsäuremorpholid | | 1,0 mg |
| Natriumchlorid | | 9,0 mg |
| Aqua pro inj. | ad | 1,0 ml |

Herstellung

Der Wirkstoff wird bei Eigen-pH in Wasser gelöst und Natriumchlorid als Isotonanz zugegeben. Die

erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 1 mg, 5 mg und 1 mg Wirkstoff.

5. Suppositorien

Jedes Zäpfchen enthält :

| | |
|---|---|
| 2-[4-(2-Chlorphenyl)-9-methyl-6H-thieno[3,2-f][1,2,4]triazolo[4,3-a][1,4]diazepin-2-yl]-ethan-1-carbonsäuremorpholid | 1,0 Teile |
| Kakaobutter (Fp. 36-37 °C) | 1 200,0 Teile |
| Carnaubawachs | 5,0 Teile |

Herstellung

Kakaobutter und Carnaubawachs werden zusammengeschmolzen. Bei 45 °C gibt man den Wirkstoff hinzu und rührt bis eine komplette Dispersion entstanden ist.

Die Mischung wird in Formen entsprechender Größe gegossen und die Zäpfchen zweckmäßig verpackt.

6. Inhalationslösungen

Zusammensetzung :

| | |
|---|---|
| a) 2-[4-(2-Chlorphenyl)-9-methyl-6H-thieno[3,2-f][1,2,4]triazolo[4,3-a][1,4]diazepin-2-yl]-ethan-1-carbonsäuremorpholid | 500 mg |
| Na-EDTA | 50 mg |
| Benzalkoniumchlorid | 25 mg |
| Natriumchlorid | 880 mg |
| Destilliertes Wasser | ad 100 ml |

Herstellung

96 % der Wassermenge werden vorgelegt, darin nacheinander Na-EDTA, Benzalkoniumchlorid, Natriumchlorid und Wirkstoff klar gelöst und mit dem restlichen Wasser aufgefüllt. Die Lösung wird in 20 ml-Tropfflaschen abgefüllt. Eine Dosis (20 Tropfen, 1 ml) enthält 5 mg Wirkstoff.

| | |
|---|---|
| b) 2-[4-(2-Chlorphenyl)-9-methyl-6H-thieno[3,2-f][1,2,4]triazolo[4,3-a][1,4]diazepin-2-yl]-ethan-1-carbonsäuremorpholid | 500 mg |
| Natriumchlorid | 820 mg |
| Destilliertes Wasser | ad 100 ml |

Herstellung

96 % der Wassermenge werden vorgelegt, darin nacheinander der Wirkstoff und Natriumchlorid gelöst, mit dem restlichen Wasser aufgefüllt und die Lösung in Eindosenbehälter (4 ml) abgefüllt. Die Lösung enthält 20 mg Wirkstoff.

**Patentansprüche**

1. Thieno-triazolo-1,4-diazepino-2-carbonsäureamide der allgemeinen Formel I

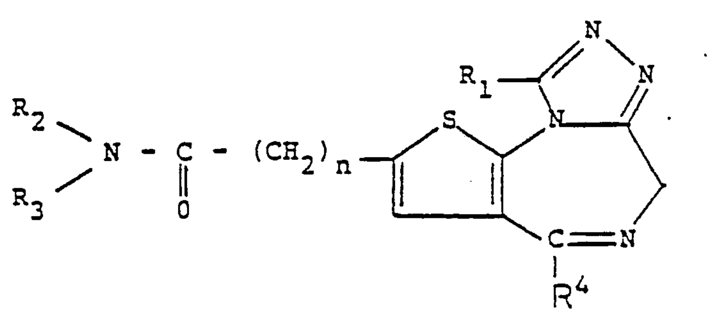

(I)

worin

$R_1$ Wasserstoff, eine geradkettige oder verzweigte Alkylgruppe mit 1-4 Kohlenstoffatomen, gegebenenfalls durch Halogen oder Hydroxy substituiert, Cyclopropyl, Alkoxy mit 1-3 Kohlenstoffatomen, Halogen ;

$R_2$ und $R_3$, die gleich oder verschieden sein können, Wasserstoff, einen geradkettigen oder verzweigten Alkyl- oder Hydroxyalkylrest mit 1-4 Kohlenstoffatomen oder beide Reste $R_2$ und $R_3$ zusammen mit dem Stickstoffatom einen 5-, 6- oder 7-Ring, der gegebenenfalls als weiteres Heteroatom ein Stickstoff-, Sauerstoff- oder Schwefelatom enthalten und wobei das zweite Stickstoffatom gegebenenfalls durch eine Alkylgruppe mit 1-4 Kohlenstoffatomen substituiert sein kann ;

$R_4$ Phenyl, wobei der Phenylring ein- oder mehrfach durch Methyl, Halogen, Nitro oder Trifluormethyl substituiert sein kann oder α-Pyridyl und

n eine der Zahlen 0, 1, 2, 3, 4, 5, 6, 7 oder 8 bedeuten.

2. Thieno-triazolo-1,4-diazepino-2-carbonsäureamide der allgemeinen Formel I gemäß Anspruch 1, worin

$R_1$, $R_2$ und $R_3$ die oben genannte Bedeutung haben,

$R_4$ Phenyl, 2-Chlorphenyl oder 2-Bromphenyl und

n die Zahl 1, 2 oder 3 bedeutet.

3. Thieno-triazolo-1,4-diazepino-2-carbonsäureamide der allgemeinen Formel I gemäß Anspruch 1, worin

$R_1$ Wasserstoff, eine geradkettige oder verzweigte Alkylgruppe mit 1-4 Kohlenstoffatomen, Cyclopropyl, Methoxy, Chlor oder Brom ;

$R_2$ und $R_3$ Wasserstoff, Methyl, Ethyl, Hydroxyethyl oder zusammen mit einem Stickstoffatom Morpholin ;

$R_4$ 2-Chlorphenyl oder 2-Bromphenyl und

n die Zahl 2 bedeutet.

4. 2-[4-(2-Chlorphenyl)-9-methyl-6H-thieno[3,2-f][1,2,4]triazolo[4,3-a][1,4]diazepin-2-yl]-ethan-1-carbonsäuremorpholid.

5. 2-[4-(2-Chlorphenyl)-9-methyl-6H-thieno[3,2-f][1,2,4]triazolo[4,3-a][1,4]diazepin-2-yl]-ethan-1-carbonsäurediethylamid.

6. Verfahren zur Herstellung von Thieno-triazolo-1,4-diazepino-2-carbonsäureamiden der allgemeinen Formel I

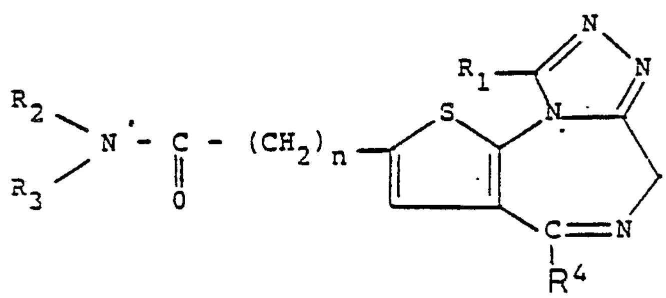

(I)

worin

$R_1$ Wasserstoff, eine geradkettige oder verzweigte Alkylgruppe mit 1-4 Kohlenstoffatomen, gegebenenfalls durch Halogen oder Hydroxy substituiert, Cyclopropyl, Alkoxy mit 1-3 Kohlenstoffatomen, Halogen ;

$R_2$ und $R_3$, die gleich oder verschieden sein können, Wasserstoff, einen geradkettigen oder verzweigten Alkyl- oder Hydroxyalkylrest mit 1-4 Kohlenstoffatomen oder beide Reste $R_2$ und $R_3$ zusammen mit dem Stickstoffatom einen 5-, 6- oder 7-Ring, der gegebenenfalls als weiteres Heteroatom ein Stickstoff-, Sauerstoff- oder Schwefelatom enthalten und wobei das zweite Stickstoffatom gegebenenfalls durch eine Alkylgruppe mit 1-4 Kohlenstoffatomen substituiert sein kann ;

$R_4$ Phenyl, wobei der Phenylring ein- oder mehrfach durch Methyl, Halogen, Nitro oder Trifluormethyl substituiert sein kann oder α-Pyridyl und

n eine der Zahlen 0, 1, 2, 3, 4, 5, 6, 7 oder 8 bedeuten,

dadurch gekennzeichnet, daß man eine entsprechende 2-Carbonsäure der allgemeinen Formel II

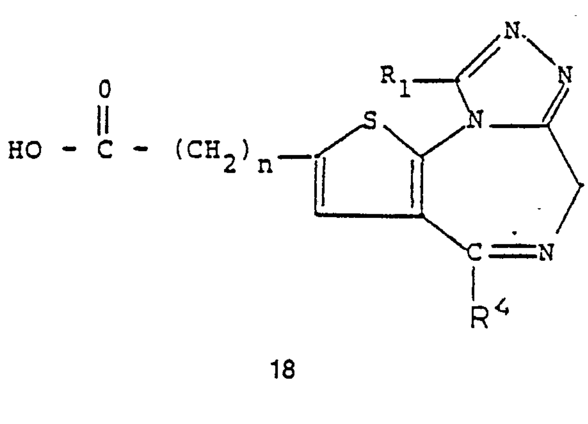

(II)

worin die Reste $R_1$ und $R_4$ die angegebene Bedeutung besitzen,

a) mit einem Amin der allgemeinen Formel III

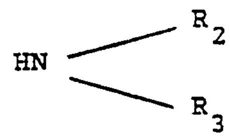

(III)

worin die Reste $R_2$ und $R_3$ die angegebene Bedeutung besitzen, umsetzt, oder daß man

b) die Carbonsäure der allgemeinen Formel II in üblicher Weise in ein Säurehalogenid oder ein Säureanhydrid überführt und dieses anschließend mit einem entsprechenden Amin der allgemeinen Formel III umsetzt.

7. Pharmazeutische Präparate, enthaltend als Wirkstoffe Verbindungen der allgemeinen Formel I in Kombination mit üblichen Hilfs- und/oder Trägerstoffen.

8. Verfahren zur Herstellung von pharmazeutischen Präparaten nach Anspruch 7, dadurch gekennzeichnet, daß man Verbindungen der allgemeinen Formel I mit üblichen galenischen Hilfs- und/oder Trägerstoffen zu üblichen pharmazeutischen Anwendungsformen verarbeitet.

9. Verbindungen der allgemeinen Formel I gemäß Anspruch 1 zur Verwendung für die Herstellung von Arzneimitteln mit PAF-antagonistischer Wirkung.

10. Verbindungen der allgemeinen Formel I gemäß Anspruch 1 zur Verwendung für die Herstellung von Arzneimitteln zur Behandlung von Asthma bronchiale.

11. Thieno-triazolo-1,4-diazepino-2-carbonsäuren der allgemeinen Formel II

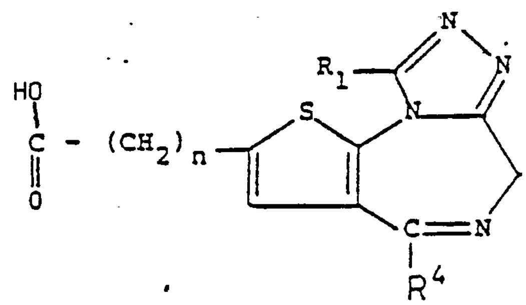

(II)

worin

$R_1$ Wasserstoff, eine geradkettige oder verzweigte Alkylgruppe mit 1-4 Kohlenstoffatomen gegebenenfalls durch Halogen oder Hydroxy substituiert, Cyclopropyl, Alkoxy mit 1-3 Kohlenstoffatomen, Halogen, und

$R_4$ Phenyl, wobei der Phenylring ein- oder mehrfach durch Methyl, Halogen, Nitro oder Trifluormethyl substituiert sein kann oder α-Pyridyl und

n eine der Zahlen 1, 2, 3, 4, 5, 6, 7 und 8 bedeuten.

12. Verfahren zur Herstellung von Thieno-triazolo-1,4-diazepino-2-carbonsäuren der allgemeinen Formel II

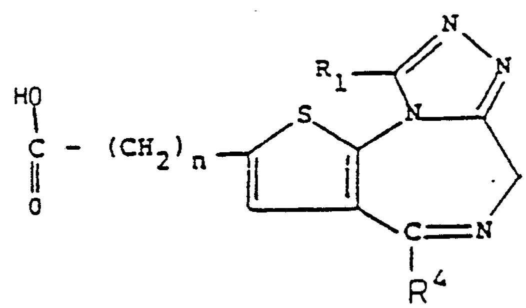

(II)

worin

$R_1$ Wasserstoff, eine geradkettige oder verzweigte Alkylgruppe mit 1-4 Kohlenstoffatomen gegebenenfalls durch Halogen oder Hydroxy substituiert, Cyclopropyl, Alkoxy mit 1-3 Kohlenstoffatomen, Halogen, und

$R_4$ Phenyl, wobei der Phenylring ein- oder mehrfach durch Methyl, Halogen, Nitro oder Trifluormethyl substituiert sein kann oder α-Pyridyl und

n eine der Zahlen 1, 2, 3, 4, 5, 6, 7 und 8 bedeuten,

dadurch gekennzeichnet, daß man

a) eine Verbindung der allgemeinen Formel

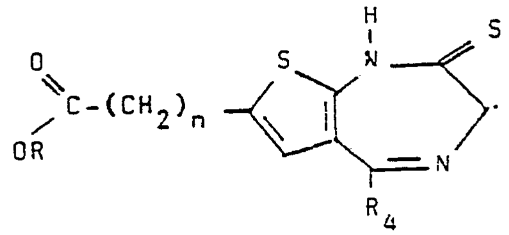

worin $R_4$ und n die zuvor genannte Bedeutung haben und R eine niedere Alkylgruppe bedeutet, mit einer Verbindung der allgemeinen Formel

$$R_1—CO—NH—NH_2$$

worin $R_1$ die oben genannte Bedeutung hat, umsetzt und anschließend verseift, oder

b) eine Verbindung der allgemeinen Formel

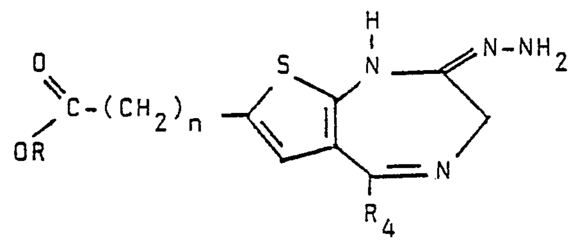

worin R, $R_4$ und n die zuvor genannte Bedeutung haben mit einer Verbindung der allgemeinen Formel

$$R_1—COCl$$

oder

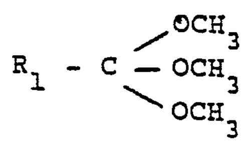

worin $R_1$ die oben genannte Bedeutung hat, umsetzt und anschließend verseift.

13. Verfahren zur Herstellung von Thieno-triazolo-1,4-diazepino-2-carbonsäuren der allgemeinen Formel II

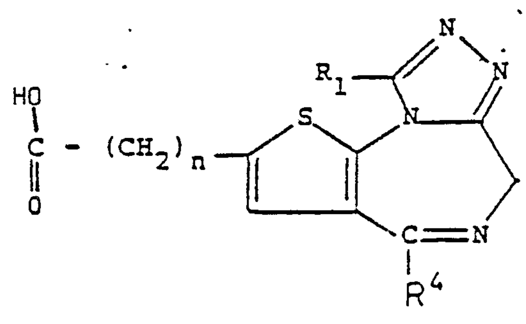

(II)

worin

$R_1$ Wasserstoff, eine geradkettige oder verzweigte Alkylgruppe mit 1-4 Kohlenstoffatomen gegebenenfalls durch Halogen oder Hydroxy substituiert, Cyclopropyl, Alkoxy mit 1-3 Kohlenstoffatomen, Halogen, und

$R_4$ Phenyl, wobei der Phenylring ein- oder mehrfach durch Methyl, Halogen, Nitro oder Trifluormethyl substituiert sein kann oder α-Pyridyl und

n eine der Zahlen 1, 2, 3, 4, 5, 6, 7 und 8 bedeuten,

dadurch gekennzeichnet, daß man eine Verbindung der allgemeinen Formel

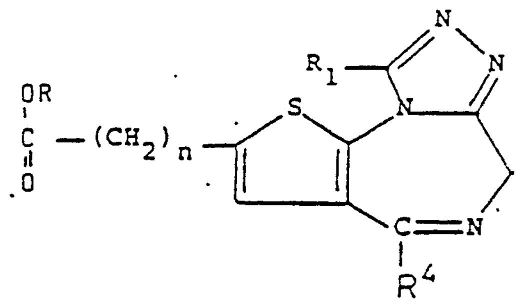

worin $R_1$, $R_4$ und n die oben genannte Bedeutung besitzen und R eine niedere Alkylgruppe bedeutet, verseift.

**Claims**

1. Thieno-triazolo-1,4-diazepino-2-carboxylic acid amides of general formula

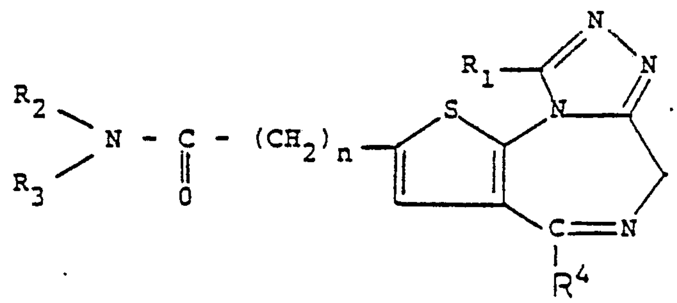

(I)

wherein

$R_1$ represents hydrogen, a straight-chained or branched alkyl group having 1 to 4 carbon atoms, optionally substituted by halogen or hydroxy, cyclopropyl, alkoxy with 1-3 carbon atoms or halogen ;

$R_2$ and $R_3$, which may be identical or different, represent hydrogen, a straight-chained or branched alkyl or hydroxyalkyl group with 1 to 4 carbon atoms or both groups $R_2$ and $R_3$ together with the nitrogen atom represent a 5-, 6- or 7-membered ring which optionally contains a nitrogen, oxygen or sulphur atom as a further heteroatom, the second nitrogen atom optionally being substituted by an alkyl group with 1-4 carbon atoms ;

$R_4$ represents phenyl, wherein the phenyl ring may be mono- or polysubstituted by methyl, halogen, nitro or trifluoromethyl, or α-pyridyl and

n represents one of the numbers 0, 1, 2, 3, 4, 5, 6, 7 or 8.

2. Thieno-triazolo-1,4-diazepino-2-carboxylic acid amides of general formula I as claimed in claim 1, wherein

$R_1$, $R_2$ and $R_3$ are defined as hereinbefore,

$R_4$ represents phenyl, 2-chlorophenyl or 2-bromophenyl and

n represents the number 1, 2 or 3.

3. Thieno-triazolo-1,4-diazepino-2-carboxylic acid amides of general formula I as claimed in claim 1 wherein

$R_1$ represents hydrogen, a straight-chained or branched alkyl group with 1 to 4 carbon atoms, cyclopropyl, methoxy, chlorine or bromine ;

$R_2$ and $R_3$ represent hydrogen, methyl, ethyl, hydroxyethyl or together with a nitrogen atom they represent morpholine ;

$R_4$ represents 2-chlorophenyl or 2-bromophenyl and

n represents the number 2.

4. 2-[4-(2-Chlorophenyl)-9-methyl-6H-thieno[3,2-f][1,2,4]triazolo[4,3-a][1,4]diazepin-2-yl]-ethane-1-carboxylic acid morpholide).

5. 2-[4-(2-Chlorophenyl)-9-methyl-6H-thieno[3,2-f][1,2,4]triazolo[4,3-a][1,4]diazepin-2-yl]-ethane-1-carboxylic acid diethylamide).

6. Process for preparing thieno-triazolo-1,4-diazepino-2-carboxylic acid amides of general formula I

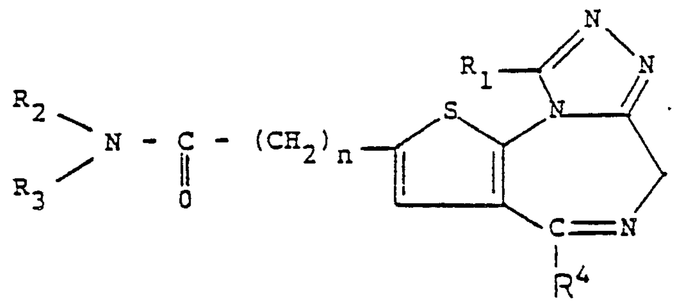

(I)

wherein

$R_1$ represents hydrogen, a straight-chained or branched alkyl group having 1 to 4 carbon atoms, optionally substituted by halogen or hydroxy, cyclopropyl, alkoxy with 1-3 carbon atoms, halogen ;

$R_2$ and $R_3$, which may be identical or different, represent hydrogen, a straight-chained or branched alkyl or hydroxyalkyl group with 1 to 4 carbon atoms or both groups $R_2$ and $R_3$ together with the nitrogen atom represent a 5-, 6- or 7-membered ring which optionally contains a nitrogen, oxygen or sulphur atom as a further heteroatom, the second nitrogen atom optionally being substituted by an alkyl group with 1-4 carbon atoms ;

$R_4$ represents phenyl, the phenyl ring optionally being mono- or polysubstituted by methyl, halogen nitro or trifluoromethyl, or α pyridyl ; and

n represents one of the numbers 0, 1, 2, 3, 4, 5, 6, 7 or 8, characterised in that a corresponding 2-carboxylic acid of general formula II

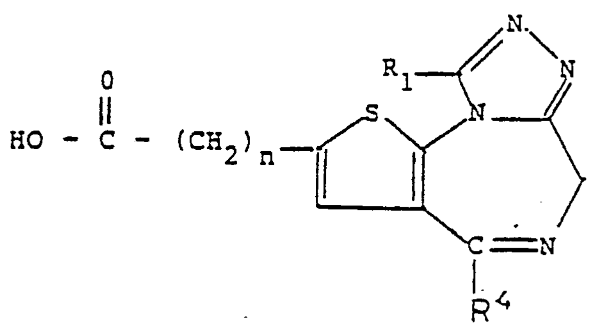

(II)

wherein the groups $R_1$ and $R_4$ are as hereinbefore defined,

a) is reacted with an amine of general formula III

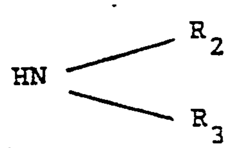

(III)

wherein the groups $R_2$ and $R_3$ are as hereinbefore defined, or

b) the carboxylic acid of general formula II is converted in the usual way into an acid halide or an acid anhydride and this is then reacted with a corresponding amine of general formula III.

7. Pharmaceutical preparations containing as active substances compounds of general formula I together with conventional excipients and/or carriers.

8. Process for preparing pharmaceutical preparations as claimed in claim 7, characterised in that compounds of general formula I are processed with conventional galenic excipients and/or carriers to form conventional pharmaceutical forms for administration.

9. Compounds of general formula I as claimed in claim 1 for use in the preparation of pharmaceutical compositions having a PAF antagonistic activity.

10. Compounds of general formula I as claimed in claim 1 for use in the preparation of pharmaceutical compositions for the treatment of bronchial asthma.

11. Thieno-triazolo-1,4-diazepino-2-carboxylic acids of general formula II

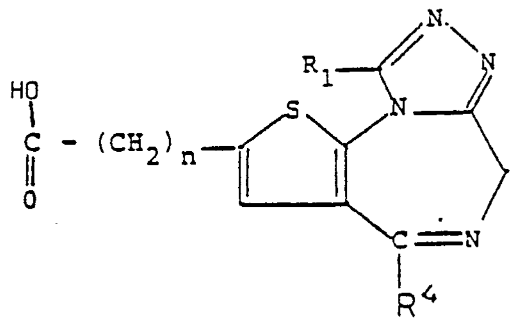

(II)

wherein

$R_1$ represents hydrogen, a straight-chained or branched alkyl group with 1-4 carbon atoms optionally substituted by halogen or hydroxy, or cyclopropyl, alkoxy with 1-3 carbon atoms, halogen, and

$R_4$ represents phenyl, the phenyl ring optionally being mono- or polysubstituted by methyl, halogen, nitro or trifluoromethyl, or α-pyridyl, and

n represents one of the numbers 1, 2, 3, 4, 5, 6, 7 and 8.

12. Process for preparing thieno-triazolo-1,4-diazepino-2-carboxylic acids of general formula II

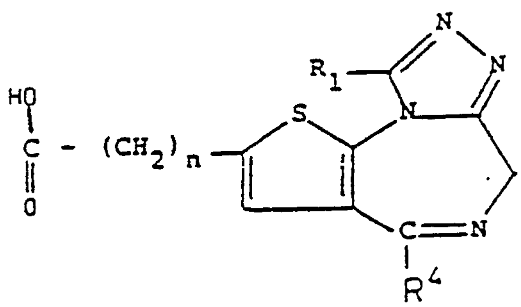

(II)

wherein

$R_1$ represents hydrogen, a straight-chained or branched alkyl group with 1-4 carbon atoms optionally substituted by halogen or hydroxy, or cyclopropyl, alkoxy with 1-3 carbon atoms, halogen, and

$R_4$ represents phenyl, in which the phenyl ring may be mono- or polysubstituted by methyl, halogen, nitro or trifluoromethyl, or α-pyridyl, and

n represents one of the numbers 1, 2, 3, 4, 5, 6, 7 and 8,

characterised in that

a) a compound of general formula

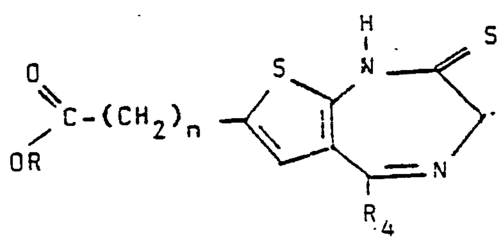

wherein $R_4$ and n are defined as hereinbefore and R represents a lower alkyl group, is reacted with a compound of general formula

$$R_1—CO—NH—NH_2$$

wherein $R_1$ is defined as hereinbefore, and subsequently saponified, or

b) a compound of general formula

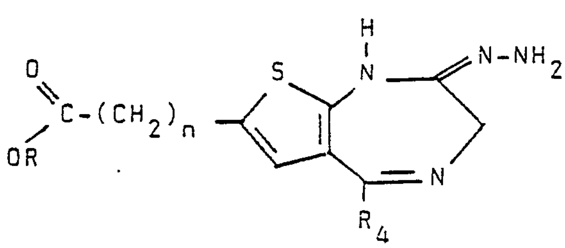

wherein R, $R_4$ and n are as hereinbefore defined, is reacted with a compound of general formula

$R_1$—COCl

or

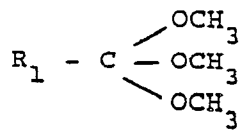

wherein $R_1$ is defined as hereinbefore, and subsequently saponified.

13. Process for preparing thieno-triazolo-1,4-diazepino-2-carboxylic acids of general formula II

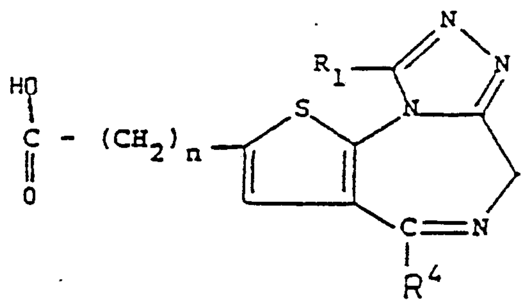

(II)

wherein

$R_1$ represents hydrogen, a straight-chained or branched alkyl group with 1-4 carbon atoms optionally substituted by halogen or hydroxy, or cyclopropyl, alkoxy with 1-3 carbon atoms, halogen, and

$R_4$ represents phenyl, the phenyl ring optionally being mono- or polysubstituted by methyl, halogen, nitro or trifluoromethyl, or α-pyridyl, and

n represents one of the numbers 1, 2, 3, 4, 5, 6, 7 and 8,

characterised in that a compound of general formula

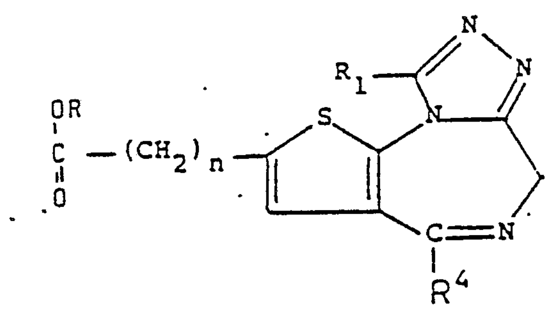

wherein $R_1$, $R_4$ and n are defined as hereinbefore and R represents a lower alkyl group, is saponified.

**Revendications**

1. Thiéno-triazolo-1,4-diazépino-2-carboxamides de formule générale I

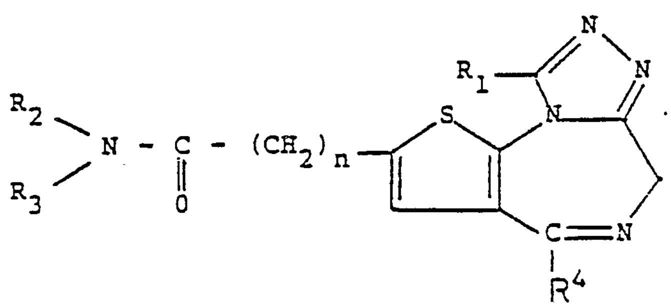

(I)

dans laquelle

$R_1$ représente l'hydrogène, un groupe alkyle à chaîne droite ou ramifiée avec 1 à 4 atomes de

carbone, éventuellement substitué par un halogène ou un hydroxyle, un cyclopropyle, un alcoxy avec 1 à 3 atomes de carbone, un halogène ;

$R_2$ et $R_3$, qui peuvent être identiques ou différents, représentent l'hydrogène, un reste alkyle ou hydroxyalkyle à chaîne droite ou ramifiée avec 1 à 4 atomes de carbone ou les deux restes $R_2$ et $R_3$ représentent, ensemble avec l'atome d'azote, un cycle penta-, hexa- ou heptagonal pouvant, le cas échéant, contenir comme autre hétéroatome un atome d'azote, d'oxygène ou de soufre, le deuxième atome d'azote pouvant éventuellement être substitué par un groupe alkyle avec 1 à 4 atomes de carbone ;

$R_4$ représente un phényle, le noyau phényle pouvant être mono- ou polysubstitué par un méthyle, un halogène, un nitro ou un trifluorométhyle, ou un α-pyridyle et

n représente un des nombres 0, 1, 2, 3, 4, 5, 6, 7 ou 8.

2. Thiéno-triazolo-1,4-diazépino-2-carboxamides de formule générale I selon la revendication 1, dans lesquels

$R_1$, $R_2$ et $R_3$ ont la signification précitée,

$R_4$ représente un phényle, un 2-chlorophényle ou un 2-bromophényle et

n représente le nombre 1, 2 ou 3.

3. Thiéno-triazolo-1,4-diazépino-2-carboxamides de formule générale I selon la revendication 1, dans lesquels

$R_1$ représente l'hydrogène, un groupe alkyle à chaîne droite ou ramifiée avec 1 à 4 atomes de carbone, un cyclopropyle, un méthoxy, du chlore ou du brome ;

$R_2$ et $R_3$ représentent l'hydrogène, un méthyle, un éthyle, un hydroxyéthyle ou, ensemble avec un atome d'azote, la morpholine ;

$R_4$ représente un 2-chlorophényle ou un 2-bromophényle et

n le nombre 2.

4. 2-[4-(2-chlorophényl)-9-méthyl-6H-thiéno[3,2-f][1,2,4]-triazolo[4,3-a][1,4]diazépine-2-yl]-éthane-1-carboxymorpholide.

5. 2-[4-(2-chlorophényl)-9-méthyl-6H-thiéno[3,2-f][1,2,4]-triazolo[4,3-a][1,4]diazépine-2-yl]-éthane-1-carboxydiéthylamide.

6. Procédé de préparation de thiéno-triazolo-1,4-diazépino-2-carboxyamide de formule générale I

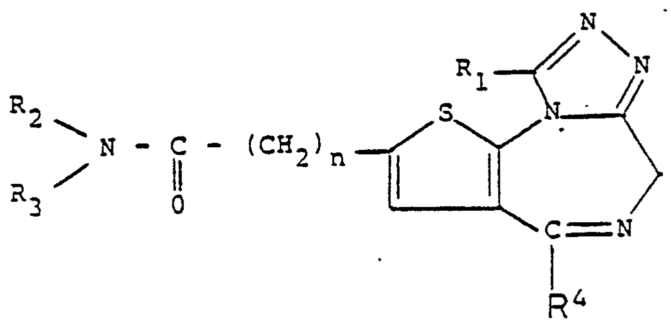

(I)

dans laquelle

$R_1$ représente l'hydrogène, un groupe alkyle à chaîne droite ou ramifiée avec 1 à 4 atomes de carbone, éventuellement substitué par un halogène ou un hydroxyle, un cyclopropyle, un alcoxy avec 1 à 3 atomes de carbone, un halogène ;

$R_2$ et $R_3$, qui peuvent être identiques ou différents, représentent l'hydrogène, un reste alkyle ou hydroxyalkyle à chaîne droite ou ramifiée avec 1 à 4 atomes de carbone ou les deux restes $R_2$ et $R_3$ représentent, ensemble avec l'atome d'azote, un cycle penta-, hexa- ou heptagonal pouvant, le cas échéant, contenir comme autre hétéroatome un atome d'azote, d'oxygène ou de soufre, le deuxième atome d'azote pouvant éventuellement être substitué par un groupe alkyle avec 1 à 4 atomes de carbone ;

$R_4$ représente un phényle, le noyau phényle pouvant être mono- ou polysubstitué par un méthyle, un halogène, un nitro ou un trifluorométhyle, ou un α-pyridyle et

n représente un des nombres 0, 1, 2, 3, 4, 5, 6, 7 ou 8, caractérisé en ce qu'on fait réagir un acide 2-carboxylique approprié de formule générale II

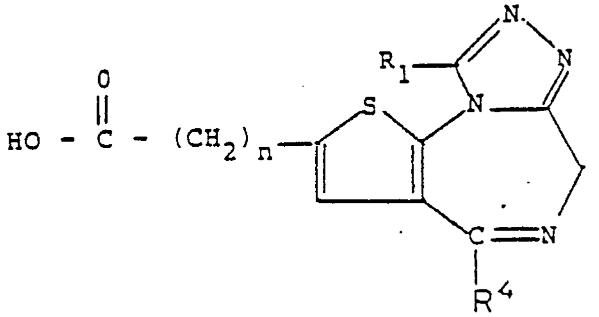

(II)

dans laquelle les restes $R_1$ et $R_4$ ont la signification indiquée,

a) avec une amine de formule générale III

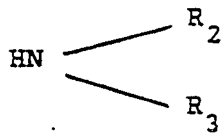

(III)

dans laquelle les restes $R_2$ et $R_3$ possèdent la signification indiquée, ou

b) qu'on transforme l'acide carboxylique de formule générale II, selon la manière habituelle, en un halogénure d'acide ou en un anhydride d'acide et fait ensuite réagir celui-ci avec une amine correspondante de formule générale III.

7. Préparations pharmaceutiques contenant, à titre d'ingrédients actifs, des composés de formule générale I en association avec les adjuvants et/ou les excipients usuels.

8. Procédé pour l'obtention de préparations pharmaceutiques selon la revendication 7, caractérisé en ce que l'on transforme des composés de formule générale I avec les adjuvants et/ou les excipients galéniques usuels en les formes d'application habituelles.

9. Composés de formule générale I selon la revendication 1 en vue de l'emploi pour la préparation de médicaments doués d'un effet antagoniste de PAF.

10. Composés de formule générale I selon la revendication 1 en vue de l'emploi pour la préparation de médicaments pour le traitement de l'asthme bronchique.

11. Acides thiéno-triazolo-1,4-diazépino-2-carboxyliques de formule générale II

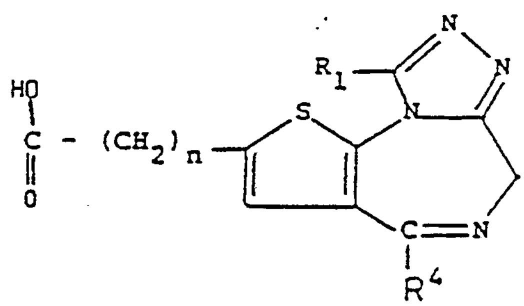

(II)

dans laquelle

$R_1$ représente l'hydrogène, un groupe alkyle à chaîne droite ou ramifiée avec 1 à 4 atomes de carbone, éventuellement substitué par un halogène ou un hydroxyle, un cyclopropyle, un alcoxy avec 1 à 3 atomes de carbone, un halogène,

$R_4$ représente un phényle, le noyau phényle pouvant être mono- ou polysubstitué par un méthyle, un halogène, un nitro ou un trifluorométhyle ou un α-pyridyle et

n représente un des nombres 1, 2, 3, 4, 5, 6, 7 et 8.

12. Procédé de préparation d'acides thiéno-triazolo-1,4-diazépino-2-carboxyliques de formule générale II

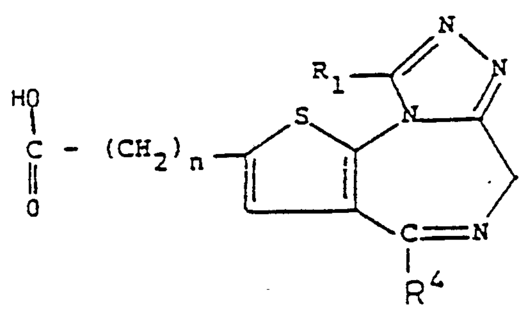

(II)

dans laquelle

$R_1$ représente l'hydrogène, un groupe alkyle à chaîne droite ou ramifiée avec 1 à 4 atomes de carbone, éventuellement substitué par un halogène ou un hydroxyle, un cyclopropyle, un alcoxy avec 1 à 3 atomes de carbone, un halogène,

$R_4$ représente un phényle, le noyau phényle pouvant être mono- ou polysubstitué par un méthyle, un halogène, un nitro ou un trifluorométhyle ou un α-pyridyle et

n représente un des nombres 1, 2, 3, 4, 5, 6, 7 et 8,

caractérisé en ce que

a) on fait réagir un composé de formule générale

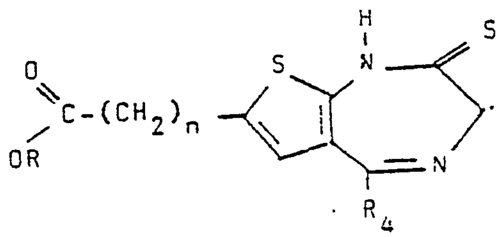

dans laquelle $R_4$ et n possèdent la signification précitée et R représente un groupe alkyle inférieur, avec un composé de formule générale

$$R_1—CO—NH—NH_2$$

où $R_1$ a la signification précédente et saponifie ensuite, ou

b) qu'on fait réagir un composé de formule générale

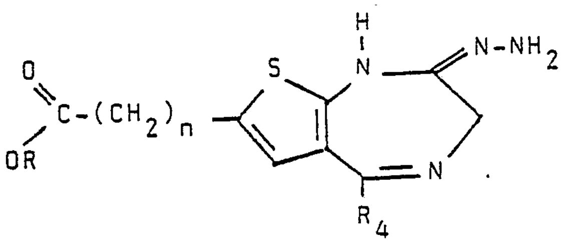

dans laquelle R, $R_4$ et n ont la signification précédente, avec un composé de formule générale

$$R_1—COCl$$

ou

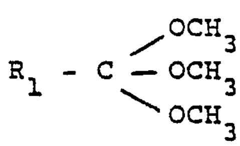

dans laquelle $R_1$ a la signification susmentionnée et saponifie ensuite.

13. Procédé de préparation d'acides thiéno-triazolo-1,4-diazépino-2-carboxyliques de formule générale II

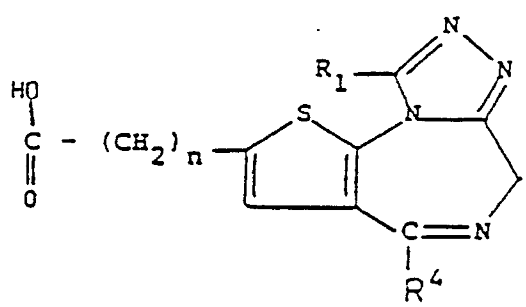

(II)

dans laquelle

$R_1$ représente l'hydrogène, un groupe alkyle à chaîne droite ou ramifiée avec 1 à 4 atomes de carbone, éventuellement substitué par un halogène ou un hydroxyle, un cyclopropyle, un alcoxy avec 1 à 3 atomes de carbone, un halogène,

$R_4$ représente un phényle, le noyau phényle pouvant être mono- ou polysubstitué par un méthyle, un halogène, un nitro ou un trifluorométhyle ou un α-pyridyle et

n représente un des nombres 1, 2, 3, 4, 5, 6, 7 et 8,

caractérisé en ce qu'on saponifie un composé de formule générale

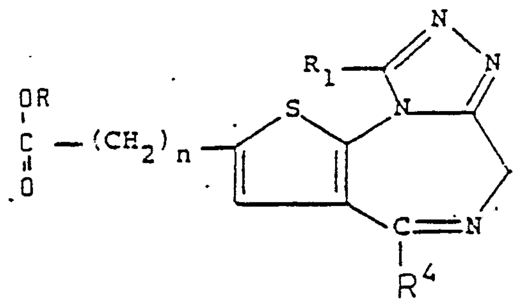

dans laquelle $R_1$, $R_4$ et n ont la signification précédente et R représente un groupe alkyle inférieur.